# EUROPEAN PATENT APPLICATION

(11) **EP 1 571 193 A1**
(43) Date of publication of application: **07.09.2005**
(21) Application number: 03778817.1
(22) Date of filing: 11.12.2003
(51) Int. Cl.: C09K 11/06, H05B 33/14, H05B 33/22

(54) **ORGANIC ELECTROLUMINESCENT DEVICE MATERIAL AND ORGANIC ELECTROLUMINESCENT DEVICE USING SAME**

(30) Priority: 12.12.2002 JP 2002360134
(71) Applicant: IDEMITSU KOSAN CO., LTD., Tokyo 100-8321 (JP)
(72) Inventor: IWAKUMA, Toshihiro, Sodegaura-shi, Chiba 299-0205 (JP); TOMITA, Seiji, Sodegaura-shi, Chiba 299-0205 (JP); ARAKANE, Takashi, Sodegaura-shi, Chiba 299-0205 (JP)
(74) Representative: Gille Struck Neidlein Prop Roos
(86) International application number: PCT/JP2003/015874
(87) International publication number: WO 2004/053019

(57) **Abstract**

A material for organic electroluminescence devices comprising a compound having a specific condensed cyclic structure having nitrogen atom and an organic electroluminescence device comprising an organic thin film layer which comprises at least one layer and is sandwiched between an anode and a cathode, wherein at least one of the layers in the organic thin film layer contains the above material, are provided. The organic electroluminescence device utilizes emission of phosphorescent light, exhibits a great current efficiency and has a long lifetime.

## Description

### TECHNICAL FIELD

The present invention relates to an organic electroluminescent ("electroluminescent" and "electroluminescence" will be referred to as "EL", hereinafter) device material and an organic EL device using the same. More particularly, the present invention relates to a material for organic EL devices which utilizes emission of phosphorescent light, exhibits a great current efficiency and has a long lifetime and an organic EL device using the material.

### BACKGROUND ART

An organic EL device is a spontaneous light emitting device which utilizes the principle that a fluorescent substance emits light by energy of recombination of holes injected from an anode and electrons injected from a cathode when an electric field is applied. Since an organic EL device of the laminate type driven under a low electric voltage was reported by C. W. Tang of Eastman Kodak Company (C. W. Tang and S. A. Vanslyke, Applied Physics Letters, Volume 51, Pages 913, 1987), many studies have been conducted on organic EL devices using organic materials as the constituting materials. Tang et al. used a laminate structure using tris(8-hydroxyquinolinol)aluminum for the light emitting layer and a triphenyldiamine derivative for the hole transporting layer. Advantages of the laminate structure are that the efficiency of hole injection into the light emitting layer can be increased, that the efficiency of forming excited particles which are formed by blocking and recombining electrons injected from the cathode can be increased, and that excited particles formed within the light emitting layer can be enclosed. As the structure of the organic EL device, a two-layered structure having a hole transporting (injecting) layer and an electron transporting and light emitting layer and a three-layered structure having a hole transporting (injecting) layer, a light emitting layer and an electron transporting (injecting) layer are well known. To increase the efficiency of recombination of injected holes and electrons in the devices of the laminate type, the structure of the device and the process for forming the device have been studied.

As the light emitting material of the organic EL device, chelate complexes such as tris(8-quinolinolato)aluminum, coumarine derivatives, tetraphenylbutadiene derivatives, bisstyrylarylene derivatives and oxadiazole derivatives are known. It is reported that light in the visible region ranging from blue light to red light can be obtained by using these light emitting materials, and development of a device exhibiting color images is expected (refer to, for example, Japanese Patent Application Laid-Open Nos. Heisei 8(1996)-239655, Heisei 7(1995)-138561 and Heisei 3(1991)-200289).

It is recently proposed that an organic phosphorescent materials is used in the light emitting layer of an organic EL device in combination with a light emitting material (for example, D. F. O'Brien, M. A. Baldo et al., "Improved energy transfer in electrophosphorescent devices", Applied Physics Letters, Vol. 74, No. 3, Pages 442 to 444, January 18, 1999; and M. A. Baldo et al., "Very high-efficiency green organic light-emitting devices based on electrophosphorescence", Applied Physics Letters, Vol. 75, No. 1, Pages 4 to 6, July 5, 1999).

As described above, a great efficiency of light emission is achieved by utilizing an organic phosphorescent material excited to the singlet state and the triplet state in the light emitting layer of an organic EL device. It is considered that singlet excimers and triplet excimers are formed in relative amounts of 1:3 due to the difference in the multiplicity of spin when electrons and holes are recombined in an organic EL device. Therefore, it is expected that an efficiency of light emission 3 to 4 times as great as that of a device utilizing fluorescence alone can be achieved by utilizing a material emitting phosphorescent light.

The organic EL device utilizing phosphorescent light emission is still under study, and an organic EL device exhibiting a great efficiency of light emssion and having a long lifetime is also being studied. As one of such studies, a device containing a phosphorescent emissive compound in the light emitting layer and emitting turquoise light at an external quantum efficiency of 10% is disclosed in Japanese Patent Application Laid-Open No. 2002-100476. However, neither the efficiency of light emission nor the luminance of the device is mentioned in the specification of Japanese Patent Application Laid-Open No. 2002-100476, and it is not known whether the device has the properties useful for practical applications. Therefore, an organic EL device utilizing the phosphorescent light emission which exhibits an efficiency of light emission and a lifetime sufficient for practical applications has been desired.

### DISCLOSURE OF THE INVENTION

The present invention has been made to overcome the above problems and has an object of providing an organic EL device which utilizes phosphorescent light emission, exhibits a great current efficiency and has a long lifetime.

As the result of extensive studies by the present inventors to achieve the above object, it was found that an organic EL device which utilized phosphorescent light emission, exhibited a great efficiency of light emission and had a long lifetime could be obtained by using a compound having a specific condensed cyclic structure having nitrogen atom. The present invention has been completed based on this knowledge.

The present invention provides a material for organic EL devices which comprises a compound represented by following general formula (1): 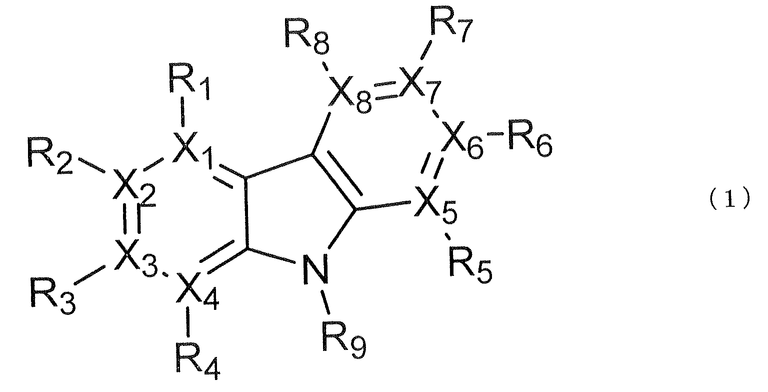 wherein X₁ to X₈ each represent carbon atom or nitrogen atom, and at least one of X₁ to X₈ represents nitrogen atom; when any of X₁ to X₈ represent carbon atom, R₁ to R₈ connected to X₁ to X₈ representing carbon atom, respectively, each represent a substituent bonded to the carbon atom, respectively, each represent a substituent bonded to the carbon atom; adjacent substituents represented by R₁ to R₈ may form a ring; when any of X₁ to X₈ represent nitrogen atom, R₁ to R₈ connected to X₁ to X₈ representing nitrogen atom, respectively, each represent a noncovalent electron pair; and R₉ represents a substituent.

The present invention also provides an organic EL device comprising a cathode, an anode and an organic thin film layer which is sandwiched between the cathode and the anode and comprises at least one layer, wherein at least one layer in the organic thin film layer contains a material for organic EL devices described above. It is preferable that a light emitting layer, an electron transporting layer and/or an electron injecting layer or a hole transporting layer and/or a hole injecting layer contains the above material for organic EL devices.

### THE MOST PREFERRED EMBODIMENT TO CARRY OUT THE INVENTION

The material for organic electroluminescence devices of the present invention comprises a compound represented by following general formula (1):

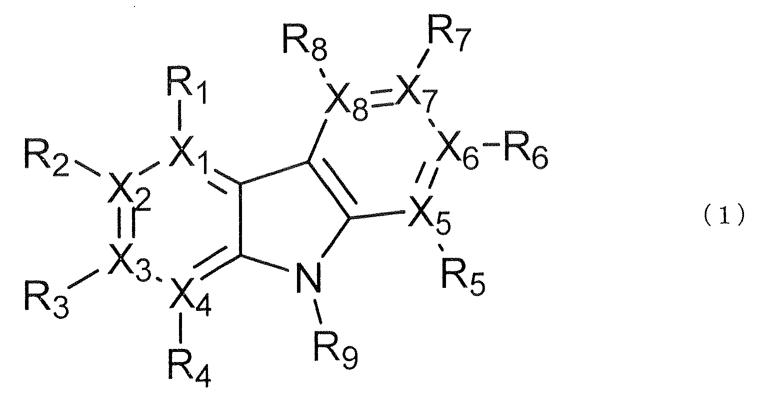

In the above formula (1), X₁ to X₈ each represent carbon atom or nitrogen atom, and at least one of X₁ to X₈ represents nitrogen atom. When any of X₁ to X₈ represent carbon atom, R₁ to R₈ connected to X₁ to X₈ representing carbon atom, respectively, each represent a substituent bonded to the carbon atom. Adjacent substituents represented by R₁ to R₈ may form a ring. When any of X₁ to X₈ represent nitrogen atom, R₁ to R₈ connected to X₁ to X₈ representing nitrogen atom, respectively, each represent a noncovalent electron pair. R₉ represents a substituent.

The substituents represented by R₁ to R₉ may each represent -L or -L-Y, in which L is directly connected to X₁ to X₈ (in the case of R₁ to R₈) or to N (in the case of R₉).

L represents hydrogen atom, a substituted or unsubstituted aryl group having 6 to 40 carbon atoms, a substituted or unsubstituted heterocyclic group having 2 to 40 carbon atoms, a substituted or unsubstituted linear or branched alkyl group having 1 to 20 carbon atoms, a substituted or unsubstituted cycloalkyl group having 6 to 40 carbon atoms, a substituted or unsubstituted amino group having 2 to 40 carbon atoms, a substituted or unsubstituted linear or branched alkoxyl group having 1 to 40 carbon atoms, a halogen atom, nitro group, a substituted or unsubstituted arylene group having 6 to 40 carbon atoms, a substituted or unsubstituted divalent heterocyclic group having 2 to 40 carbon atoms, a linear or branched substituted or unsubstituted alkylene group having 1 to 20 carbon atoms or a substituted or unsubstituted cycloalkylene group having 6 to 40 carbon atoms.

Y represents hydrogen atom, a substituted or unsubstituted aryl group having 6 to 40 carbon atoms, a substituted or unsubstituted heterocyclic group having 2 to 40 carbon atoms, a substituted or unsubstituted linear or branched alkyl group having 1 to 20 carbon atoms, a substituted or unsubstituted cycloalkyl group having 6 to 40 carbon atoms, a substituted or unsubstituted amino group having 2 to 40 carbon atoms, a substituted or unsubstituted linear or branched alkoxyl group having 1 to 40 carbon atoms, a halogen atom or nitro group.

Examples of the aryl group represented by L include phenyl group, 1-naphthyl group, 2-naphthyl group, 1-anthryl group, 2-anthryl group, 9-anthryl group, 1-phenanthryl group, 2-phenanthryl group, 3-phenanthryl group, 4-phenanthryl group, 9-phenanthryl group, 1-naphthacenyl group, 2-naphthacenyl group, 9-naphthacenyl group, 1-pyrenyl group, 2-pyrenyl group, 4-pyrenyl group, 2-biphenylyl group, 3-biphenylyl group, 4-biphenylyl group, p-terphenyl-4-yl group, p-terphenyl-3-yl group, p-terphenyl-2-yl group, m-terphenyl-4-yl group, m-terphenyl-3-yl group, m-terphenyl-2-yl group, o-tolyl group, m-tolyl group, p-tolyl group, p-t-butylphenyl group, p-(2-phenylpropyl)phenyl group, 3-methyl-2-naphthyl group, 4-methyl-1-naphthyl group, 4-methyl-1-anthryl group, 4'-methylbiphenylyl group, 4"-t-butyl-p-terphenyl-4-yl group, fluorenyl group and perfluoroaryl groups.

Examples of the alkyl group represented by L include methyl group, trifluoromethyl group, ethyl group, propyl group, isopropyl group, n-butyl group, s-butyl group, isobutyl group, t-butyl group, n-pentyl group, n-hexyl group, n-heptyl group, n-octyl group, hydroxymethyl group, 1-hydroxyethyl group, 2-hydroxyethyl group, 2-hydroxyisobutyl group, 1,2-dihydroxyethyl group, 1,3-dihydroxyisopropyl group, 2,3-dihydroxy-t-butyl group, 1,2,3-trihydroxypropyl group, chloromethyl group, 1-chloroethyl group, 2-chloroethyl group, 2-chloroisobutyl group, 1,2-dichloroethyl group, 1,3-dichloroisopropyl group, 2,3-dichloro-t-butyl group, 1,2,3- trichloropropyl group, bromomethyl group, 1-bromoethyl group, 2-bromoethyl group, 2-bromoisobutyl group, 1,2-dibromoethyl group, 1,3-dibromoisopropyl group, 2,3-dibromo-t-butyl group, 1,2,3-tribromopropyl group, iodomethyl group, 1-iodoethyl group, 2-iodoethyl group, 2-iodoisobutyl group, 1,2-diiodoethyl group, 1,3-diiodoisopropyl group, 2,3-diiodo-t-butyl group, 1,2,3-triiodopropyl group, aminomethyl group, 1-aminoethyl group, 2-aminoethyl group, 2-aminoisobutyl group, 1,2-diaminoethyl group, 1,3-diaminoisopropyl group, 2,3-diamino-t-butyl group, 1,2,3-triaminopropyl group, cyanomethyl group, 1-cyanoethyl group, 2-cyanoethyl group, 2-cyanoisobutyl group, 1,2-dicyanoethyl group, 1,3-dicyanoisopropyl group, 2,3-dicyano-t-butyl group, 1,2,3- tricyanopropyl group, nitromethyl group, 1-nitroethyl group, 2-nitroethyl group, 2-nitroisobutyl group, 1,2-dinitroethyl group, 1,3-dinitroisopropyl group, 2,3-dinitro-t-butyl group and 1,2,3-trinitropropyl group.

As for the substituted aryl group, when, for example, phenyl group having 6 carbon atoms is substituted with a substituent such as phenyl group and methyl group, examples of the substituted aryl group include groups having the following structures:

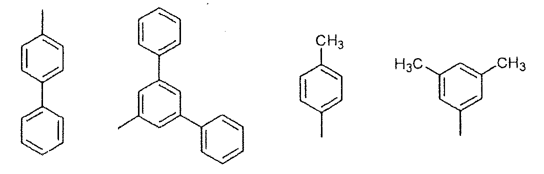

Examples of the cycloalkyl group represented by L include cyclopentyl group, cyclohexyl group, 4-methylcyclohexyl group, adamantyl group and norbornyl group.

Example of the amino group represented by L include dimethylamino group, methylethylamino group, diphenylamino group, diisopropylamino group, bis-diphenylamino group, carbazolyl group, diethylamino group, ditolylamino group, indolyl group, piperidyl group and pyrrolidinyl group.

The alkoxyl group represented by L is represented by -OY¹. Examples of the group represented by Y¹ include methyl group, trifluoromethyl group, ethyl group, propyl group, isopropyl group, n-butyl group, s-butyl group, isobutyl group, t-butyl group, n-pentyl group, n-hexyl group, n-heptyl group, n-octyl group, hydroxymethyl group, 1-hydroxyethyl group, 2-hydroxyethyl group, 2-hydroxyisobutyl group, 1,2-dihydroxyethyl group, 1,3-dihydroxy-isopropyl group, 2,3-dihydroxy-t-butyl group, 1,2,3- trihydroxypropyl group, chloromethyl group, 1-chloroethyl group, 2-chloroethyl group, 2-chloroisobutyl group, 1,2-dichloroethyl group, 1,3-dichloroisopropyl group, 2,3-dichloro-t-butyl group, 1,2,3- trichloropropyl group, bromomethyl group, 1-bromoethyl group, 2-bromoethyl group, 2-bromoisobutyl group, 1,2-dibromoethyl group, 1,3-dibromolsopropyl group, 2,3-dibromo-t-butyl group, 1,2,3-tribromopropyl group, iodomethyl group, 1-iodoethyl group, 2-iodoethyl group, 2-iodoisobutyl group, 1,2-diiodoethyl group, 1,3-diiodoisopropyl group, 2,3-diiodo-t-butyl group, 1,2,3-triiodopropyl group, aminomethyl group, 1-aminoethyl group, 2-aminoethyl group, 2-aminoisobutyl group, 1,2-diaminoethyl group, 1,3-diaminoisopropyl group, 2,3-diamino-t-butyl group, 1,2,3-triaminopropyl group, cyanomethyl group, 1-cyanoethyl group, 2-cyanoethyl group, 2-cyanoisobutyl group, 1,2-dicyanoethyl group, 1,3-dicyanoisopropyl group, 2,3-dicyano-t-butyl group, 1,2,3-tricyanopropyl group, nitromethyl group, 1-nitroethyl group, 2-nitroethyl group, 2-nitroisobutyl group, 1,2-dinitroethyl group, 1,3-dinitroisopropyl group, 2,3-dinitro-t-butyl group and 1,2,3-trinitropropyl group.

Examples of the halogen atom represented by L include fluorine atom, chlorine atom, bromine atom and iodine atom.

Examples of the arylene group represented by L include divalent groups derived from the groups described above as the examples of the aryl group represented by L.

As for the substituted aryl group, when, for example, phenyl group having 6 carbon atoms is substituted with a substituent such as phenyl group and methyl group, examples of the substituted aryl group include groups having the following structures:

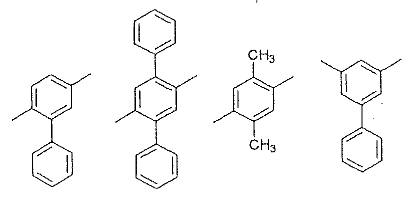

Examples of the substituted or unsubstituted divalent heterocyclic group having 2 to 40 carbon atoms which is represented by L include divalent groups derived from the groups described above as the examples of the heterocyclic group represented by L.

Examples of the alkylene group represented by L include divalent groups derived from the groups described above as the examples of the alkyl group represented by L.

Examples of the cycloalkylene group represented by L include divalent groups derived from the groups described above as the examples of the cycloalkyl group represented by L.

Examples of the aryl group, the heterocyclic group, the alkyl group, the cycloalkyl group, the amino group, the alkoxyl group and the halogen group represented by Y include the groups described above as the examples of the corresponding groups represented by L.

It is preferable that, in general formula (1), one to three among X₁ to X₈ each represent nitrogen atom, and the others each represent carbon atom. It is more preferable that X₃ and/or X₆ represents nitrogen atom, and the others each represent carbon atom.

It is still more preferable that at least one of R₁ to R₈ represents β-carbolinyl group. In other words, L and/or Y represents β-carbolinyl group.

Examples of the group substituting hydrogen atom on the atoms represented by X₁ to X₈ and in the substituents represented by R₁ to R₉ include halogen atoms such as fluorine atom, chlorine atom and bromine atom, cyano group, silyl group, amino group, aryl groups, aryloxyl groups, heterocyclic group, alkyl groups, alkoxyl groups, aralkyl groups and cycloalkyl groups.

Examples of the compound represented by general formula (1) comprised in the material for organic EL devices of the present invention are shown in the following. However, the material of the present invention is not limited to the compounds shown as the examples.

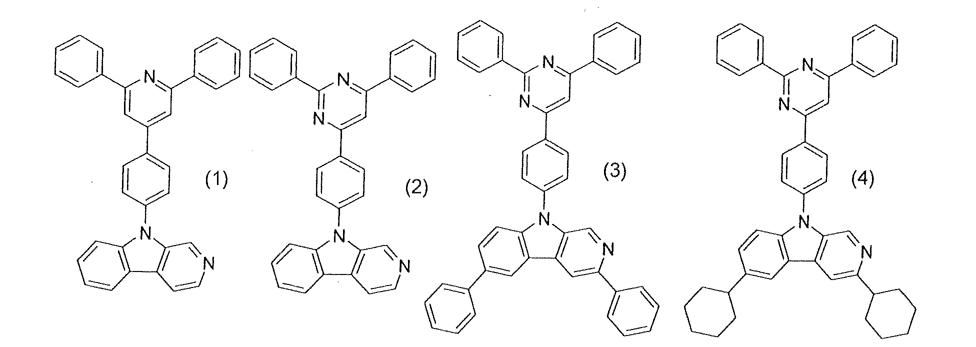

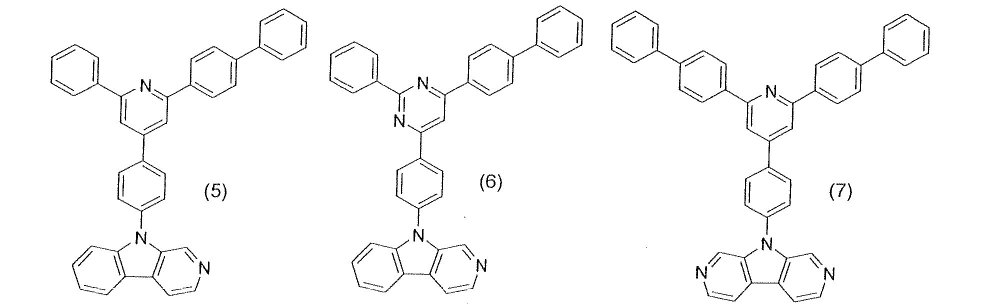

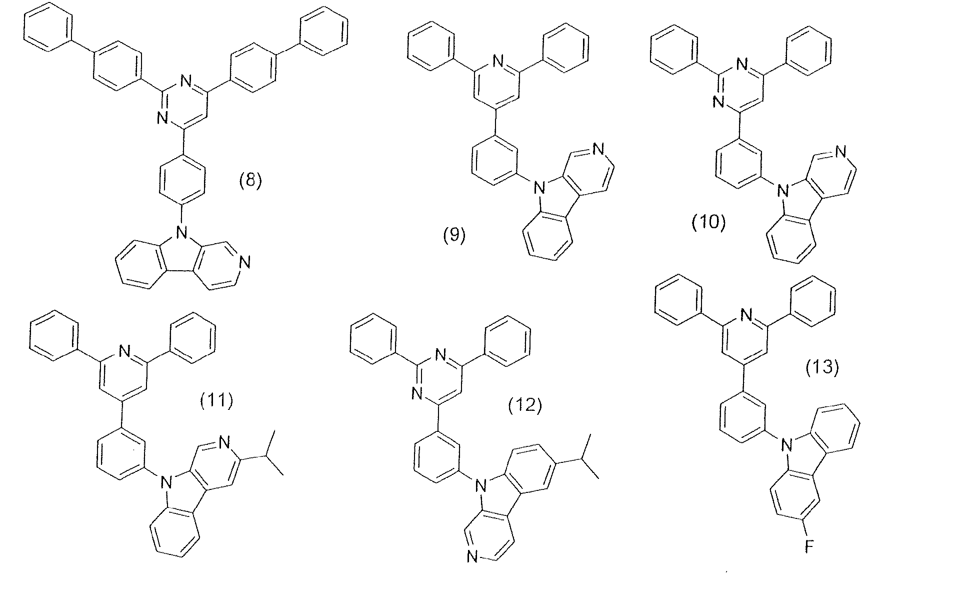

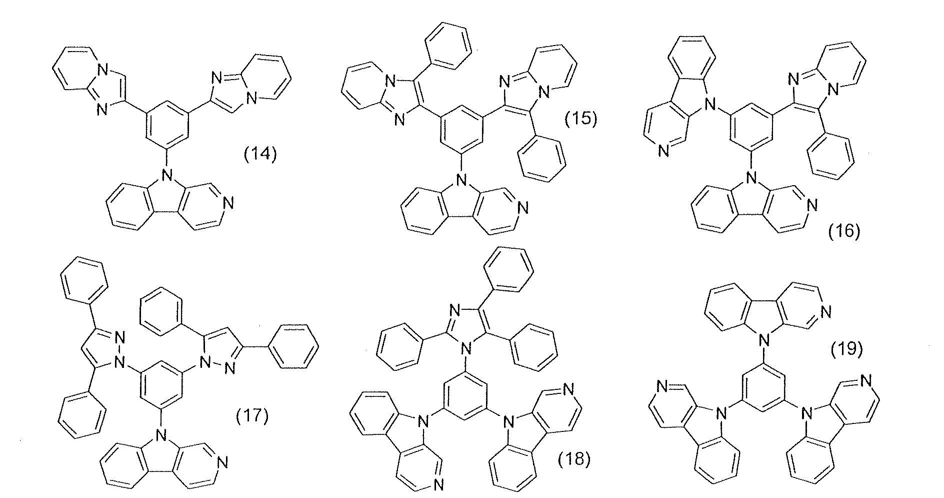

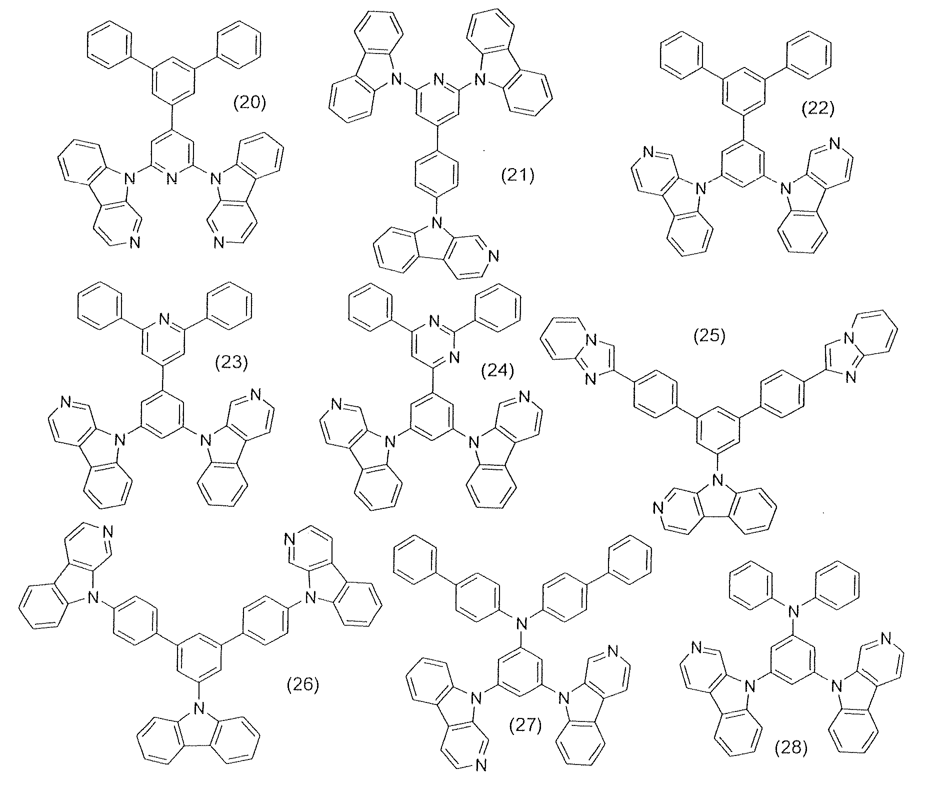

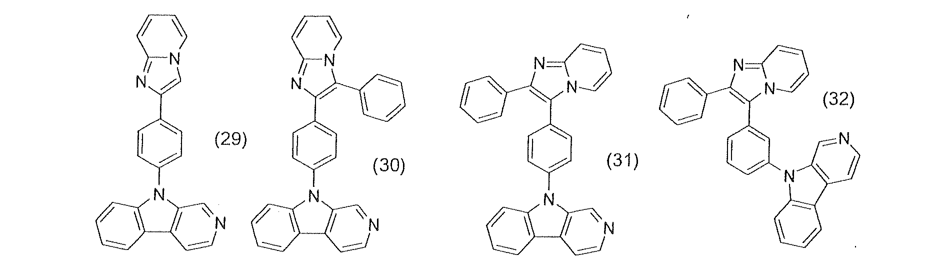

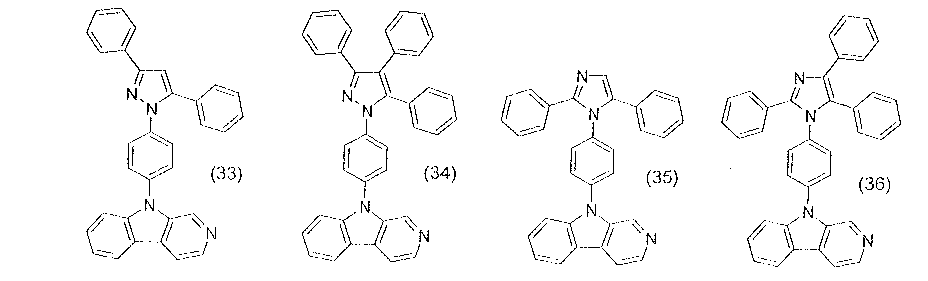

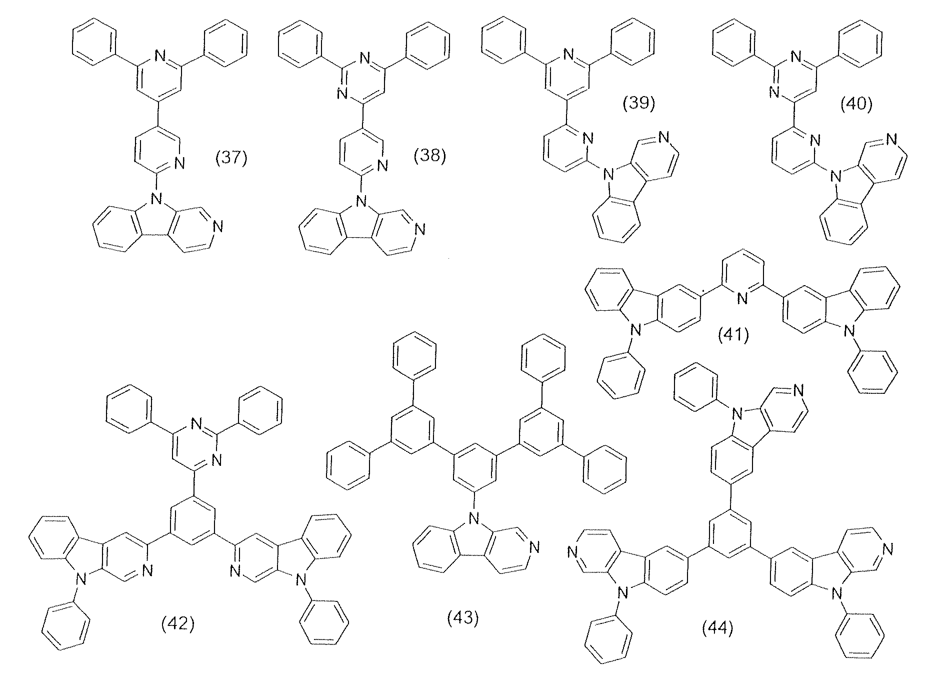

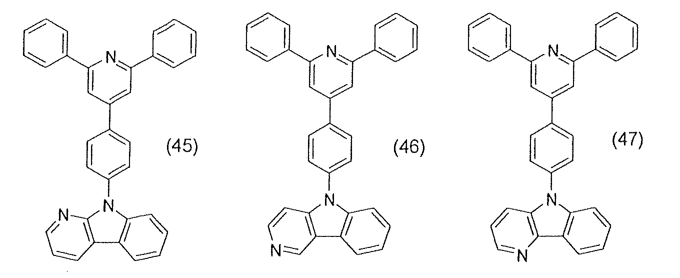

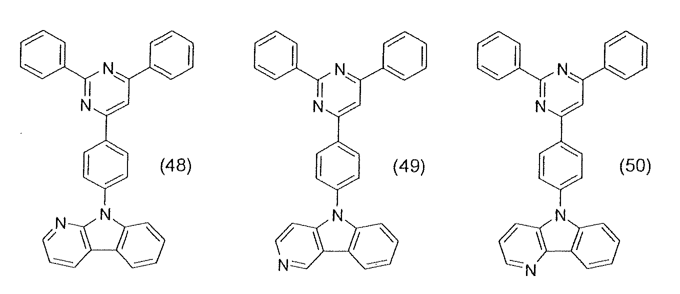

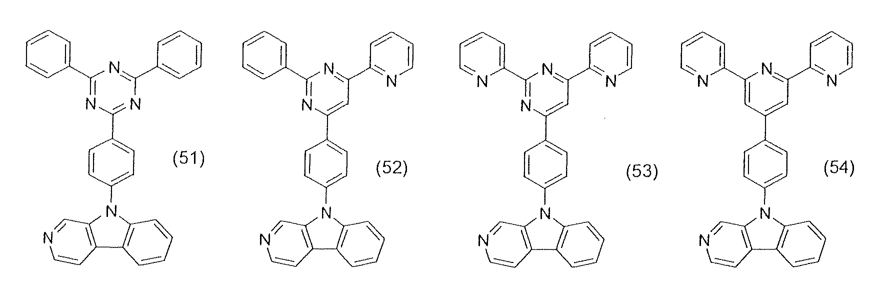

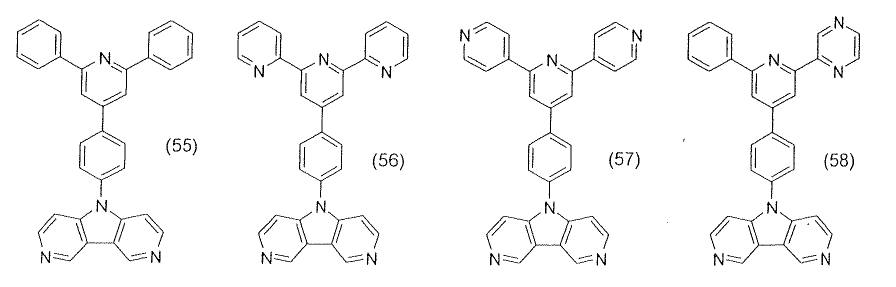

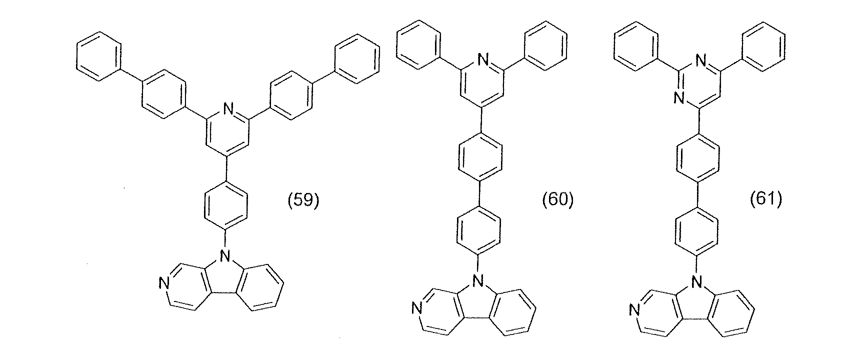

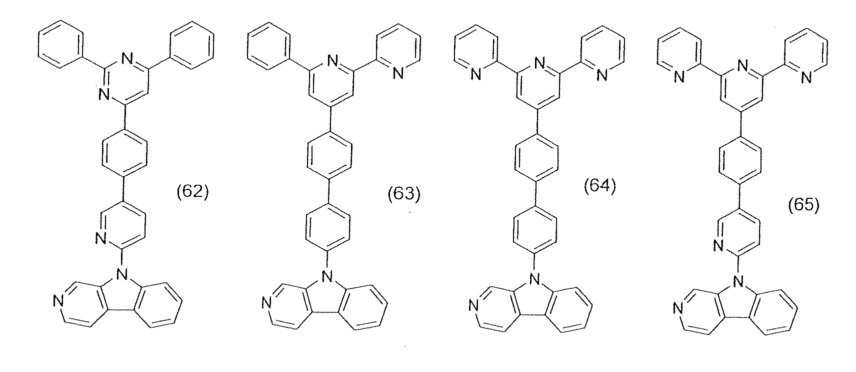

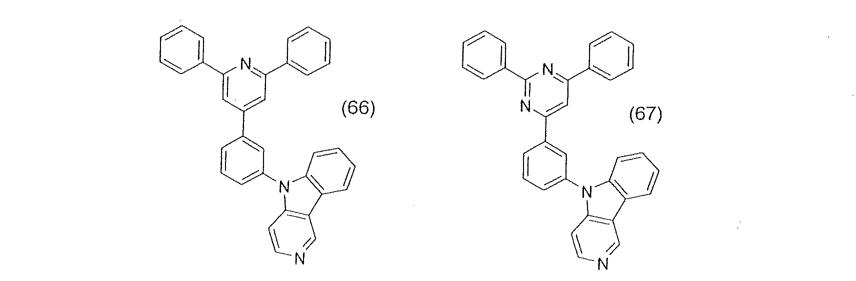

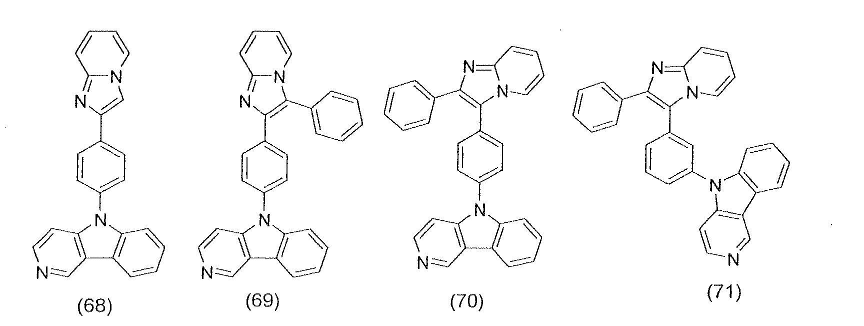

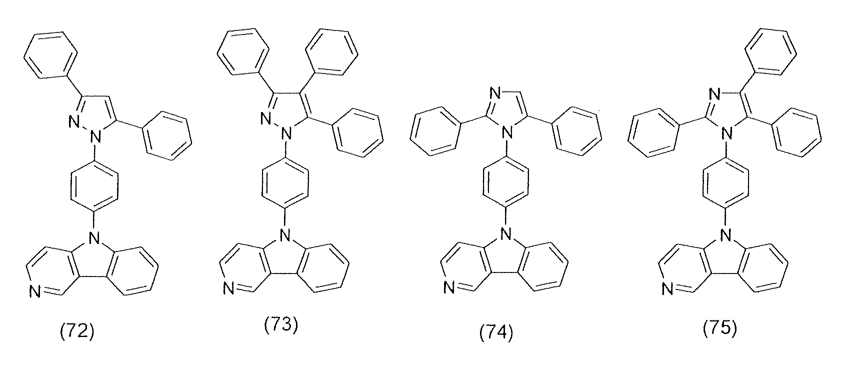

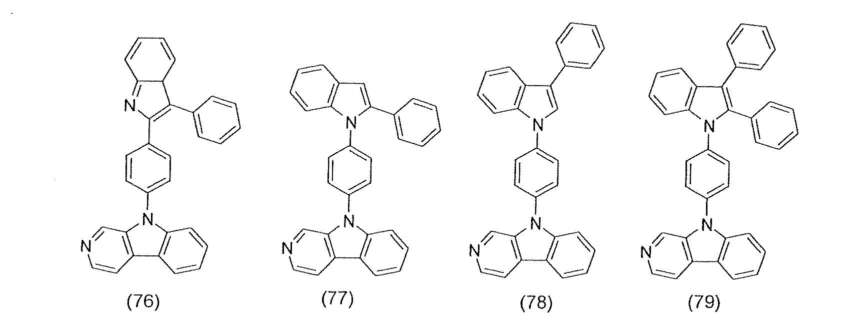

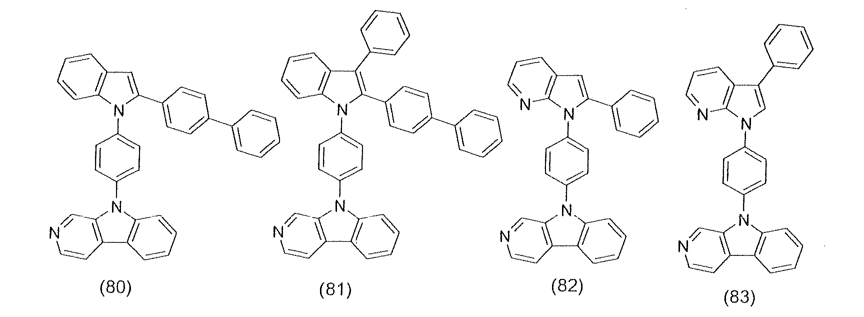

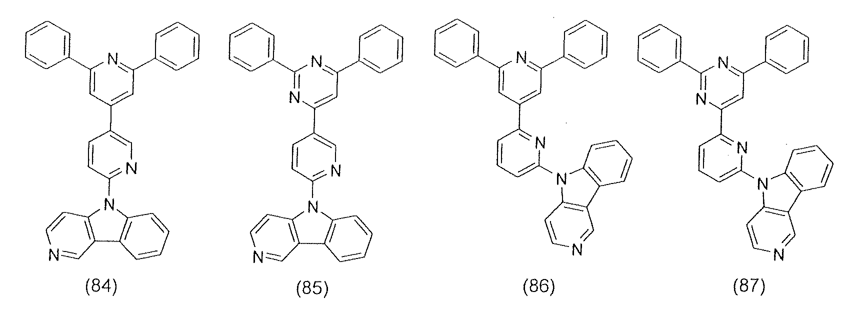

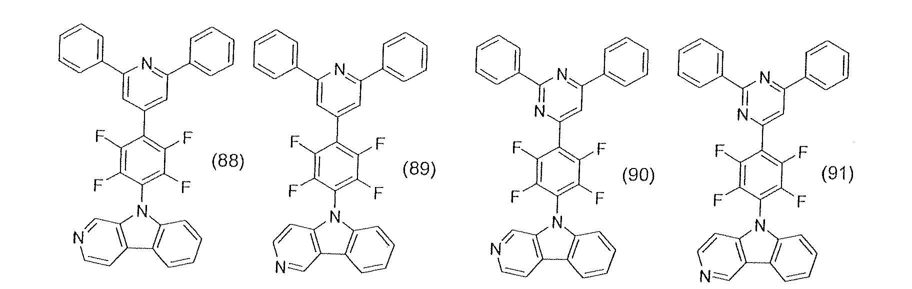

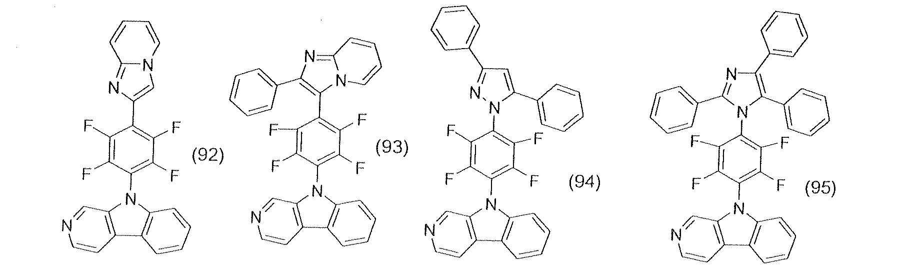

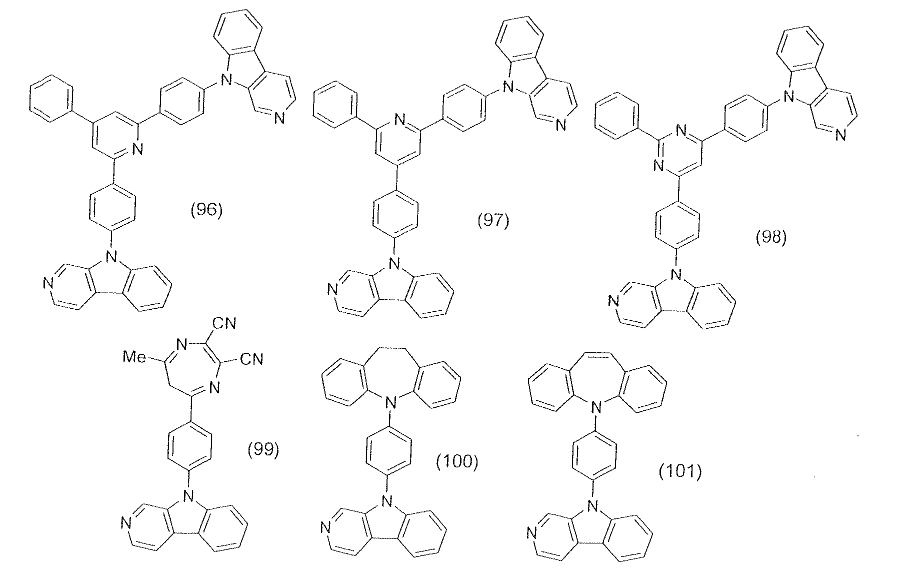

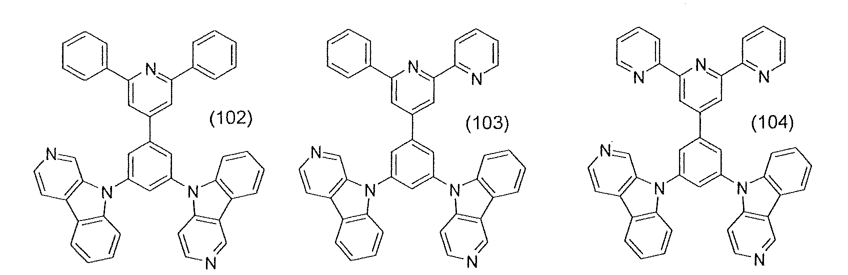

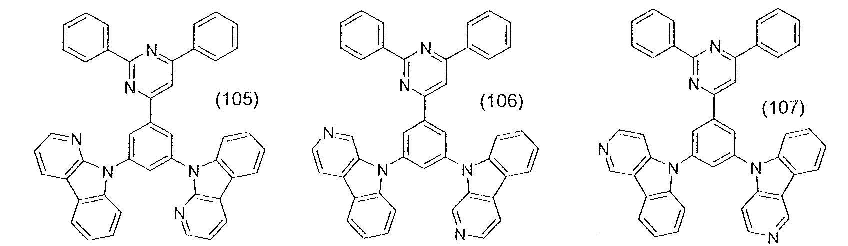

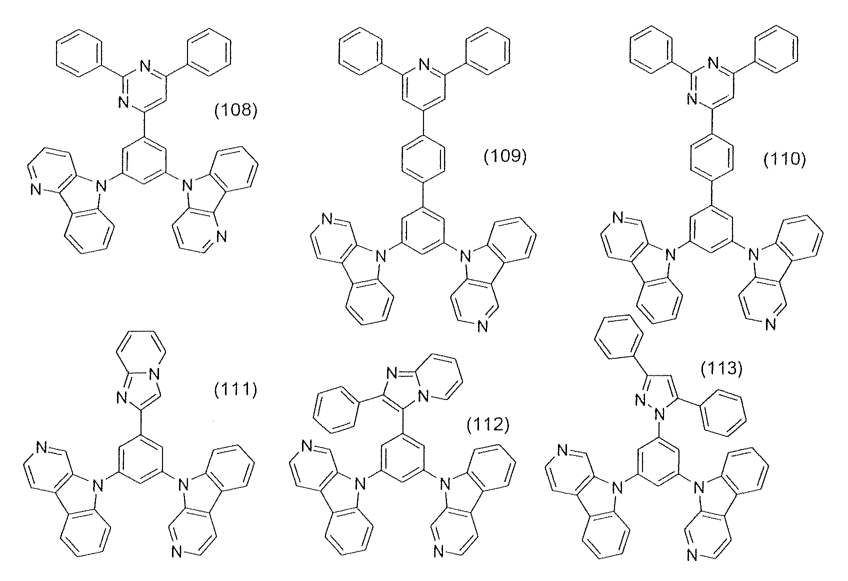

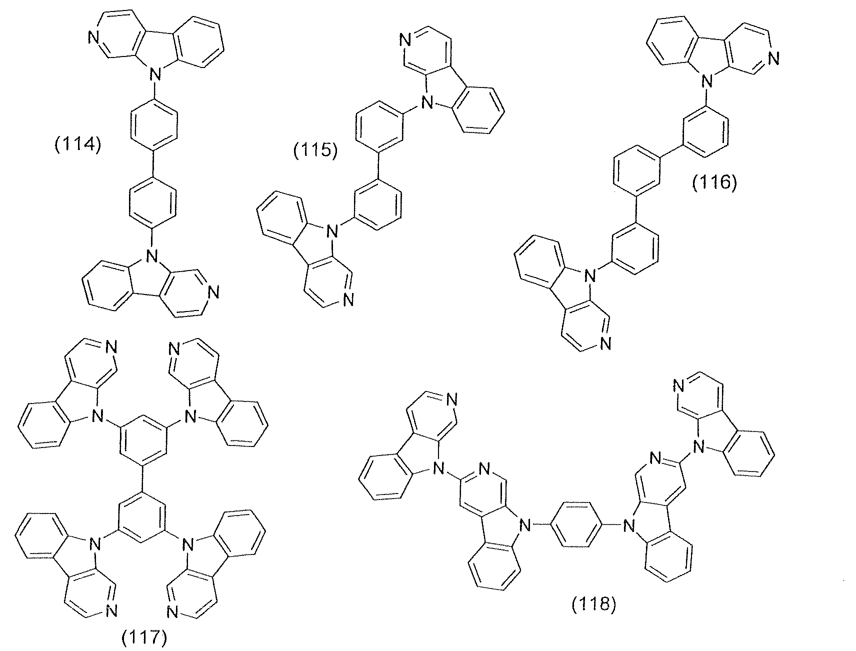

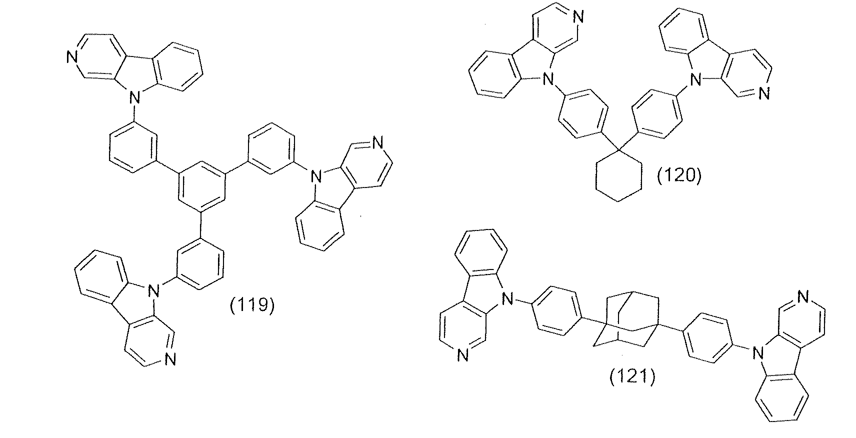

It is preferable that the material for organic EL devices of the present invention comprising the compound represented by general formula (1) has an energy gap of the triplet state of 2.5 to 3.3 eV and more preferably 2.6 to 3.2 eV.

It is preferable that the material for organic EL devices of the present invention comprising the compound represented by general formula (1) has an energy gap of the singlet state of 2.8 to 3.8 eV and more preferably 2.9 to 3.6 eV.

The organic EL device comprises a cathode, an anode and an organic thin film layer which is sandwiched between the cathode and the anode and comprises at least one layer, and at least one layer in the organic thin film layer contains the material for organic EL devices comprising the material represented by general formula (1) described above.

It is preferable that the light emitting layer, the electron transporting layer and/or the electron injecting layer or the hole transporting layer an/or the hole injecting layer in the organic EL device of the present invention contains the material for organic EL devices comprising the material represented by general formula (1) described above.

It is preferable that the organic thin film layer in the organic EL device of the present invention contains a phosphorescent emissive compound. As the phosphorescent emissive compound, metal complexes emitting light by a multiplet excitation which is the excitation to the triplet state or higher are preferable. Examples of the metal complex include the following compounds:

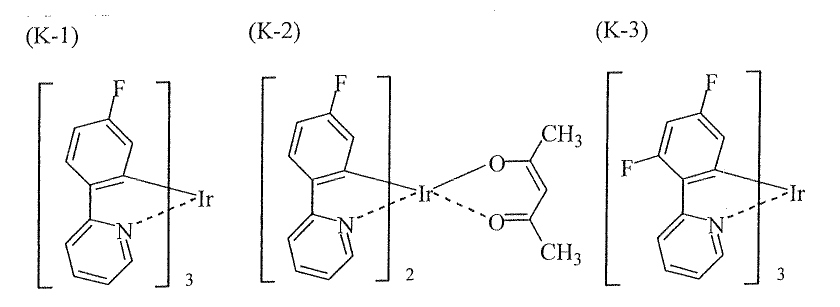

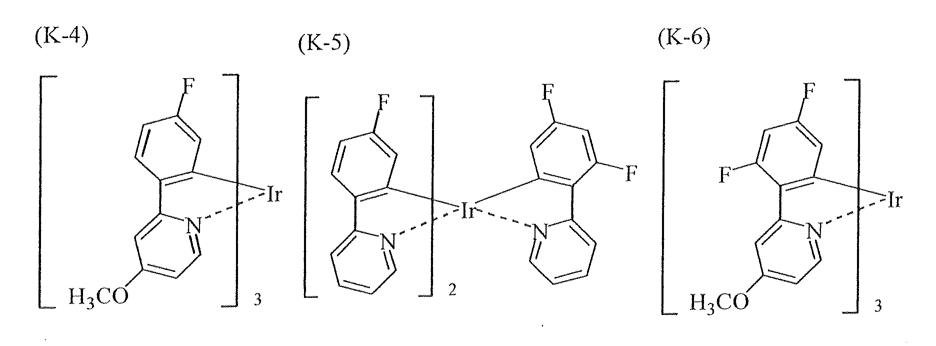

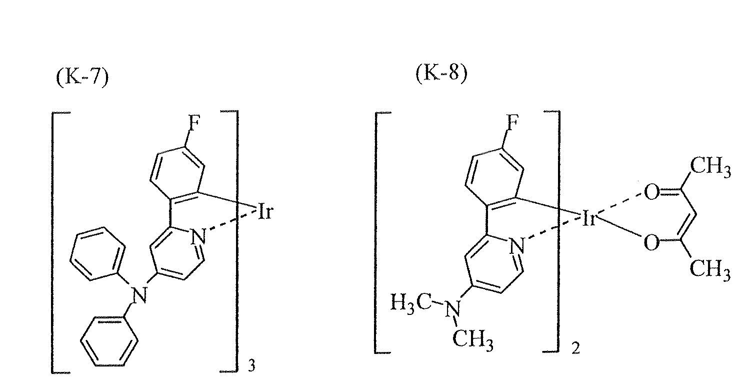

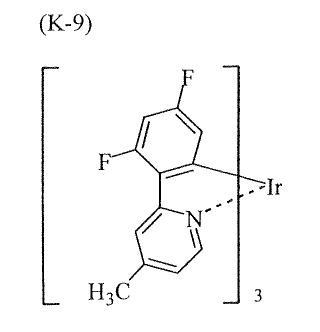

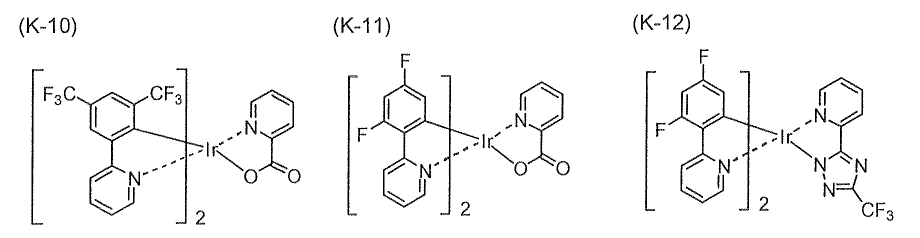

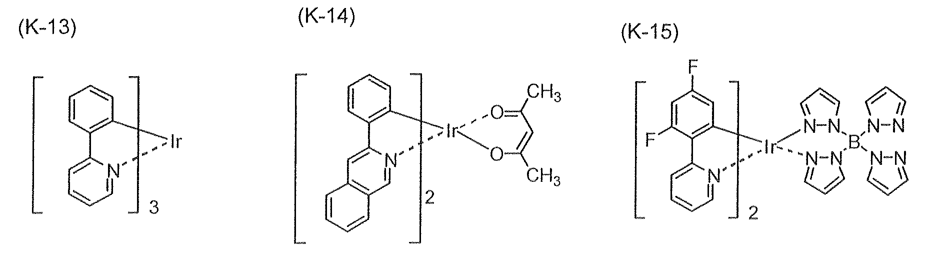

It is preferable that the material for organic EL devices is a host material of the organic EL device. The host material is a material into which holes and electrons can be injected and which has the function of transporting holes and electrons and emitting fluorescence by recombination of holes and electrons.

The compound represented by general formula (1) in the present invention is useful also as the organic host material for phosphorescence devices since the energy gap of the singlet state is as great as 2.8 to 3.8 eV and the energy gap of the triplet state is as great as 2.5 to 3.3 eV.

The phosphorescence device is an organic device which comprises a substance emitting light based on the transition from the energy level in the triplet state to the energy level in the ground singlet state with a stronger intensity than those emitted from other substances, examples of which include phosphorescent substances such as organometallic complexes comprising at least one metal selected from Groups 7 to 11 of the Periodic Table, and emits light under an electric field utilizing the so-called phosphorescence.

In the light emitting layer of the organic EL device, in general, the singlet exciton and the triplet exciton are contained in the formed excited molecules as a mixture, and it is said that the triplet exciton is formed in a greater amount such that the ratio of the amount of the singlet exciton to the amount of the triplet exciton is 1:3. In conventional organic EL devices using the fluorescence, the exciton contributing to the light emission is the singlet exciton, and the triplet exciton does not emit light. Therefore, the triplet exciton is ultimately consumed as heat, and the light is emitted by the singlet exciton which is formed in a smaller amount. Therefore, in these organic EL devices, the energy transferred to the triplet exciton causes a great loss in the energy generated by the recombination of holes and electrons.

In contrast, it is considered that, by using the material of the present invention for the phosphorescence device, the efficiency of light emission three times as great as that of a device using fluorescence can be obtained since the triplet exciton can be used for the emission of light. It is also considered that, when the compound of the present invention is used for the light emitting layer of the phosphorescence device, an excited triplet level in an energy state higher than the excited triplet level of a phosphorescent organometallic complex comprising a metal selected from the Group 7 to 11 of the Periodic Table contained in the layer, is achieved; the film having a more stable form is provided; the glass transition temperature is higher (Tg: 80 to 160°C); holes and/or electrons are efficiently transported; the compound is electrochemically and chemically stable; and the formation of impurities which may work as a trap or cause loss in the light emission is suppressed during the preparation and the use.

The organic EL device of the present invention comprises, as described above, a cathode, an anode and an organic thin film layer comprising at least one layer and sandwiched between the cathode and the anode. When the organic thin film layer comprises a single layer, a light emitting layer is formed between the anode and the cathode. The light emitting layer contains a light emitting material and may further contain a hole injecting material for transporting holes injected from the anode to the light emitting material or an electron injecting material for transporting electrons injected from the cathode to the light emitting material. It is preferable that the light emitting material exhibits a very excellent quantum yield, has a great ability of transporting both holes and electrons and forms a uniform thin layer. Examples of the organic EL device of the multi-layer type include organic EL devices comprising a laminate having a multi-layer structure such as (the anode / the hole injecting layer / the light emitting layer / the cathode), (the anode / the light emitting layer / the electron injecting layer / the cathode) and (the anode / the hole injecting layer / the light emitting layer / the electron injecting layer / the cathode).

For the light emitting layer, in addition to the material comprising the compound represented by general formula (1) of the present invention, conventional host materials, light emitting materials, doping materials, hole transporting materials and electron transporting materials and combinations of these materials may be used in combination, where necessary. By using a multi-layer structure for the organic EL device, decreases in the luminance and the lifetime due to quenching can be prevented, and the luminance of emitted light and the efficiency of light emission can be improved with other doping materials. By using other doping materials contributing to the light emission of the phosphorescent light in combination, the luminance of emitted light and the efficiency of light emission can be improved in comparison with those of conventional devices.

In the organic EL device of the present invention, the hole transporting layer, the light emitting layer and the electron transporting layer may each have a multi-layer structure. When the hole transporting layer has a multi-layer structure, the layer into which holes are injected from the electrode is called the hole injecting layer, and the layer which receives holes from the hole injecting layer and transports holes to the light emitting layer is called the hole transporting layer. Similarly, when the electron transporting layer has a multi-layer structure, the layer into which electron are injected from the electrode is called the electron injecting layer, and the layer which receives electrons from the electron injecting layer and transports electrons to the light emitting layer is called the electron transporting layer. The layers are selected in accordance with various properties such as the energy levels of the material, heat resistance and adhesion with the organic thin film layers and the metal electrodes.

In the organic EL device of the present invention, the electron transporting layer and the hole transporting layer may contain the material for organic EL devices of the present invention which comprises the compound represented by general formula (1). The hole injecting layer, the electron injecting layer and the hole barrier layer may contain the material for organic EL devices of the present invention. The phosphorescent emissive compounds and the material for organic EL devices of the present invention may be used together as a mixture.

Examples of the light emitting material and the host material which can be used for the organic thin film layer in combination with the compound represented by general formula (1) include anthracene, naphthalene, phenanthrene, pyrene, tetracene, coronen, chrysene, fluoresceine, perylene, phthaloperylene, naphthaloperylene, perynone, phthaloperynone, naphthaloperynone, diphenylbutadiene, tetraphenylbutadiene, coumarine, oxadiazole, aldazine, bisbenzoxazoline, bisstyryl, pyrazine, cyclopentadiene, metal complexes of quinoline, metal complexes of aminoquinoline, metal complexes of benzoquinoline, imines, diphenylethylene, vinylanthracene, diaminoanthracene, diaminocarbazole, pyrane, thiopyrane, polymethine, melocyanine, oxinoid compounds chelated with imidazole, quinacridone, rubrene, stilbene-based derivatives and fluorescent pigments. However, the light emitting material and the host material are not limited to the compounds described above.

As the hole injecting material, compounds which have the ability to transport holes, exhibit the excellent effect of receiving holes injected from the anode and the excellent effect of injecting holes to the light emitting layer or the light emitting material, prevent transfer of excitons formed in the light emitting layer to the electron injecting layer or the electron injecting material and have the excellent ability of forming a thin film, are preferable. Examples of the hole injecting compound include phthalocyanine derivatives, naphthalocyanine derivatives, porphyrin derivatives, oxazoles, oxadiazoles, triazoles, imidazoles, imidazolones, imidazolethiones, pyrazolines, pyrazolones, tetrahydroimidazoles, oxazoles, oxadiazoles, hydrazones, acylhydrazones, polyarylalkanes, stilbene, butadiene, triphenylamine of the benzidine type, triphenylamine of the styrylamine type, triphenylamine of the diamine type, derivatives of the above compounds and macromolecular materials such as polyvinylcarbazoles, polysilanes and electrically conductive macromolecules. However, the hole injecting material is not limited to these materials.

Among these hole injecting materials, the more effective hole injecting materials are aromatic tertiary amine derivatives and phthalocyanine derivatives. Examples of the aromatic tertiary amine derivative include triphenylamine, tritolylamine, tolyldiphenylamine, N,N'-diphenyl-N,N'-(3-methylphenyl)-1,1'-biphenyl-4,4'-diamine, N,N, N',N'-(4-methylphenyl)-1,1'-phenyl-4,4'-diamine, N,N,N',N'-(4-methylphenyl)-1,1'-biphenyl-4,4'-diamine, N,N'-diphenyl-N,N'-dinaphthyl-1,1'-biphenyl-4,4'-diamine, N,N'-(methylphenyl)-N,N'-(4-n-butylphenyl)-phenanthrene-9,10-diamine, N,N-bis(4-di-4-tolylaminophenyl)-4-phenylcyclohexane and oligomers and polymers having the skeleton structure of these aromatic tertiary amines. However, the aromatic tertiary amine is not limited to these compounds. Examples of the phthalocyanine (Pc) derivative include phthalocyanine derivatives and naphthalocyanine derivatives such as H₂-Pc, CuPc, CoPc, NiPc, ZnPc, PdPc, FePc, MnPc, ClAlPc, ClGaPc, ClInPc, ClSnPc, Cl₂SiPc, (HO)AlPc, (HO)GaPc, VOPc, TiOPc, MoOPc and GaPc-O-GaPc. However the phthalocyanine derivative is not limited to these compounds.

As the electron injecting material, compounds which have the ability to transport electrons, exhibit the excellent effect of receiving electrons injected from the anode and the excellent effect of injecting electrons to the light emitting layer or the light emitting material, prevent transfer of excitons formed in the light emitting layer to the hole injecting layer and have the excellent ability of forming a thin film, are preferable. Examples of the electron injecting compound include fluorenone, anthraquinodimethane, diphenoquinone, thiopyrane dioxide, oxazoles, oxadiazoles, triazoles, imidazoles, perylenetetracarboxylic acid, quinoxaline, fluorenylidenemethane, anthraquinodimethane, anthrone and derivatives of these compounds. However, the electron injecting material is not limited to these compounds.

Among these electron injecting materials, the more effective electron injecting materials are metal complex compounds and five-membered derivatives having nitrogen atom. Examples of the metal complex compound include 8-hydroxyquinolinatolithium, bis(8-hydroxy-quinolinato)zinc, bis(8-hydroxyquinolinato)copper, bis(8-hydroxy-quinolinato)manganese, tris(8-hydroxyquinolinato)aluminum, tris(2-methyl-8-hydroxyquinolinato)aluminum, tris(8-hydroxyquinolinato)-gallium, bis(10-hydroxybenzo[h]quinolinato)beryllium, bis(10-hydroxy-benzo[h]quinolinato)zinc, bis(2-methyl-8-quinolinato)chlorogallium, bis(2-methyl-8-quinolinato)(o-cresolato)gallium, bis(2-methyl-8-quinolinato)-(1-naphtholato)aluminum and bis(2-methyl-8-quinolinato)(2-naphtholato)-gallium. However the electron injecting material is not limited to these compounds.

As the five-membered derivative having nitrogen atom, oxazoles, thiazoles, oxadiazoles, thiadiazoles, triazoles and derivatives of these compounds are preferable. Examples of the five-membered derivative having nitrogen atom include 2,5-bis(1-phenyl)-1,3,4-oxazole, dimethylPOPOP, 2,5-bis(1-phenyl)-1,3,4-thiazole, 2,5-bis(1-phenyl)-1,3,4-oxadiazole, 2-(4'-tert-butylphenyl)-5-(4"-biphenyl)-1,3,4-oxadiazole, 2,5-bis(1-naphthyl)-1,3,4-oxadiazole, 1,4-bis[2-(5-phenyloxadiazolyl)]benzene, 1,4-bis[2-(5-phenyloxadiazolyl)-4-tert-butylbenzene], 2-(4'-tert-butylphenyl)-5-(4"-biphenyl)-1,3,4-thiadiazole, 2,5-bis(1-naphthyl)-1,3,4-thiadiazole, 1,4-bis[2-(5-phenylthiadiazolyl)]benzene, 2-(4'-tert-butylphenyl)-5-(4"-biphenyl)-1,3,4-triazole, 2,5-bis(1-naphthyl)-1,3,4-triazole and 1,4-bis[2-(5-phenyltriazolyl)]benzene. However, the five-membered derivative having nitrogen atom is not limited to these compounds.

The property of charge injection can be improved by adding an electron-accepting compound to the hole injecting material and an electron-donating compound to the electron injecting material.

As the electrically conductive material used for the anode of the organic EL device of the present invention, a material having a work function greater than 4 eV is suitable, and carbon, aluminum, vanadium, iron, cobalt, nickel, tungsten, silver, gold, platinum, palladium, alloys of these metals, metal oxides such as tin oxides and indium oxide used for ITO substrates and NESA substrates and organic electrically conductive resins such as polythiophene and polypyrrol are used. As the electrically conductive material used for the cathode, a material having a work function smaller than 4 eV is suitable, and magnesium, calcium, tin, lead, titanium, yttrium, lithium, ruthenium, manganese, aluminum and alloys of these metals are used. However, the electrically conductive material used for the cathode is not limited to these materials. Typical examples of the alloy include magnesium/silver, magnesium/indium and lithium/aluminum. However, the alloy is not limited to these alloys. The composition of the alloy is controlled by the temperature of the source of vaporization, the atmosphere and the degree of vacuum, and a suitable composition is selected. The anode and the cathode may be formed with a structure having two or more layers, where necessary.

The organic EL device of the present invention may comprise an inorganic compound layer between at least one of the electrodes and the above organic thin film layer. Examples of the inorganic compound used for the inorganic compound layer include various types of oxides, nitrides and oxide nitrides such as alkali metal oxides, alkaline earth metal oxides, rare earth oxides, alkali metal halides, alkaline earth metal halides, rare earth halides, SiOₓ, AlOₓ, SiNₓ, SiON, AlON, GeOₓ, LiOₓ, LiON, TiOₓ, TiON, TaOₓ, TaON, TaNₓ and C. In particular, as the component contacting the anode, SiOₓ, AlOₓ, SiNₓ, SiON, AlON, GeOₓ and C are preferable since a stable interface layer of injection is formed. As the component contacting the cathode, LiF, MgF₂, CaF₂, MgF₂ and NaF are preferable.

In the organic EL device of the present invention, it is preferable that at least one surface is sufficiently transparent in the region of the wavelength of the light emitted by the device so that the light emission is achieved efficiently. It is preferable that the substrate is also transparent.

For the transparent electrode, the conditions in the vapor deposition or the sputtering are set so that the prescribed transparency is surely obtained using the above electrically conductive material. It is preferable that the electrode of the light emitting surface has a transmittance of light of 10% or greater. The substrate is not particularly limited as long as the substrate has the mechanical and thermal strength and is transparent. Examples of the substrate include glass substrates and transparent films of resins. Examples of the transparent film of a resin include films of polyethylene, ethylene-vinyl acetate copolymers, ethylene-vinyl alcohol copolymers, polypropylene, polystyrene, polymethyl methacrylate, polyvinyl chloride, polyvinyl alcohol, polyvinyl butyral, nylon, polyether ether ketones, polysulfones, polyether sulfones, tetrafluoroethylene-perfluoroalkyl vinyl ether copolymers, polyvinyl fluoride, tetrafluoroethylene-ethylene copolymers, tetrafluoroethylene-hexafluoropropylene copolymers, polychlorotrifluoroethylene, polyvinylidene fluoride, polyesters, polycarbonates, polyurethanes, polyimides, polyether imides, polyimides and polypropylene.

In the organic EL device of the present invention, it is possible that a protective layer is formed on the surface of the device or the entire device is covered with silicone oil or a resin so that stability under the effect of the temperature, the humidity and the atmosphere is improved.

For the formation of each layer of the organic EL device of the present invention, any of the dry processes of film formation such as the vacuum vapor deposition, the sputtering, the plasma plating and the ion plating and the wet processes of film formation such as the spin coating, the dipping and the flow coating, can be conducted. The thickness of each film is not particularly limited. However, it is necessary that the thickness of the film be set at a suitable value. When the thickness is excessively great, application of a greater voltage is necessary to obtain the same output of the light, and the current efficiency decreases. When the thickness is excessively small, pin holes are formed, and sufficient light emission cannot be obtained even when an electric field is applied. In general, a thickness in the range of 5 nm to 10 µm is suitable and a thickness in the range of 10 nm to 0.2 µm is preferable.

When the wet process of film formation is used, the material forming each layer is dissolved or suspended in a suitable solvent such as ethanol, chloroform, tetrahydrofuran and dioxane, and a thin film is formed from the obtained solution or suspension. Any of the above solvents can be used. For any of the layers, suitable resins and additives may be used to improve the property for film formation and to prevent formation of pin holes in the film. Examples of the resin which can be used include insulating resins such as polystyrene, polycarbonates, polyarylates, polyesters, polyamides, polyurethanes, polysulfones, polymethyl methacrylate, polymethyl acrylate, cellulose and copolymer resins derived from these resins; photoconductive resins such as poly-N-vinylcarbazole and polysilanes; and electrically conductive resins such as polythiophene and polypyrrol. Examples of the additive include antioxidants, ultraviolet light absorbents and plasticizers.

As described above, by using the compound represented by general formula (1) for the organic thin film layer of the organic EL device of the present invention, the organic EL device emitting blue light with a high purity of color can be obtained. This organic EL device can be advantageously used for a photosensitive member for electronic photograph, a planar light emitting member such as a flat panel display of wall televisions, a back light of copiers, printers and liquid crystal displays, a light source for instruments, a display panel, a marking light and an accessory.

The present invention will be described more specifically with reference to examples in the following. However, the present invention is not limited to the examples.

### EXAMPLE

The triplet energy gap and the singlet energy of a compound were measured in accordance with the following methods.

### (1) Measurement of the triplet energy

The lowest excited triplet energy level T1 was measured. The phosphorescence spectrum of a sample was measured (a 10 µmoles/liter EPA solution (diethyl ether : isopentane : ethanol=5:5:2 by volume); 77K; a quartz cell; FLUOROLOG II manufactured by SPEX Company). A tangent was drawn to the increasing line at the short wavelength side of the phosphorescence spectrum, and the wavelength (the end of light emission) at the intersection of the tangent and the abscissa was obtained. The obtained wavelength was converted into the energy.

### (2) Measurement of the singlet energy

The excited singlet energy was measured. Using a toluene solution (10⁻⁵ moles/liter) of a sample, the absorption spectrum was obtained by a spectrometer for absorption of ultraviolet and visible light manufactured by HITACHI Co. Ltd. A tangent was drawn to the increasing line at the long wavelength side of the spectrum, and the wavelength (the end of absorption) at the intersection of the tangent and the abscissa was obtained. The obtained wavelength was converted into the energy.

### Synthesis Example 1 (Synthesis of Compound (1))

The route of synthesis of Compound (1) is shown in the following.

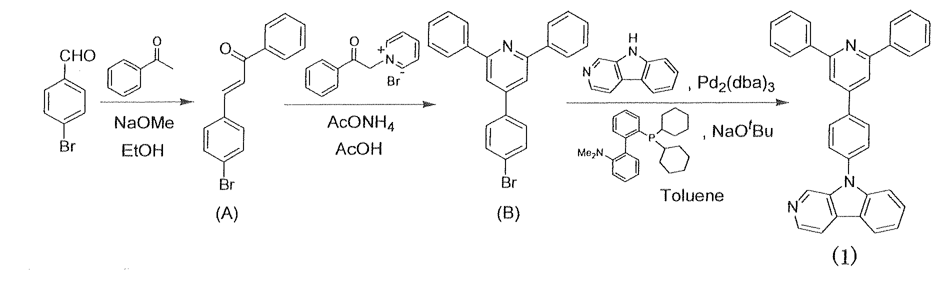

### (1) Synthesis of intermediate product (A)

Into 300 ml of ethanol, 15.0 g (81 mmole) of 4-bromobenzaldehyde and 9.7 g (81 mmole) of acetophenone were dissolved. To the resultant solution, 16.6 ml (81 mmole) of a 28% solution of sodium methoxide in methanol was added, and the obtained mixture was stirred at the room temperature for 9 hours. After the reaction was completed, the formed crystals were separated by filtration and washed with ethanol, and 19.6 g (the yield: 84%) of intermediate product (A) was obtained.

### (2) Synthesis of intermediate product (B)

Into 27 ml of acetic acid, 9.0 g (31 mmole) of intermediate product (A), 8.7 g (31 mmole) of 1-phenacylpyridinium bromide and 19.3 g (250 mmole) of ammonium acetate were suspended, and the resultant suspension was heated under the refluxing condition for 12 hours. The reaction fluid was cooled to the room temperature. Toluene and water were added to the fluid, and the resultant mixed fluid was separated into two layers. The organic layer was washed with a 10% aqueous solution of sodium hydroxide and a saturated aqueous solution of sodium chloride, successively, and dried with anhydrous sodium sulfate. After the organic solvent was removed by distillation under a reduced pressure, 27 ml of ethanol was added to the residue. The formed crystals were separated by filtration and washed with ethanol, and 10.6 g (the yield: 88%) of intermediate product (B) was obtained.

### (3) Synthesis of Compound (1)

Into 15 ml of toluene, 3.0 g (8 mmole) of intermediate product (B), 1.4 g (8 mmole) of β-carboline, 0.18 g (0.2 mmole) of tris(dibenzylideneacetone)dipalladium, 0.23 g (0.6 mmole) of 2-dicyclohexylphosphino-2'-(N,N-dimethylamino)biphenyl and 1.0 g (11 mmole) of sodium tert-butoxide were suspended, and the resultant suspension was heated under the refluxing condition for 20 hours under the atmosphere of argon. The reaction fluid was cooled to the room temperature. Methylene chloride and water were added to the fluid, and the resultant mixture was separated into two layers. The organic layer was washed with water and dried with anhydrous sodium sulfate. After the organic solvent was removed by distillation under a reduced pressure, the obtained residue was purified in accordance with the silica gel column chromatography, and 1.7 g (the yield: 46%) of crystals were obtained.

It was confirmed in accordance with 90 MHz ¹H-NMR and the filed desorption mass spectroscopy (FD-MS) that the obtained crystals were the object compound. The result of the measurement in accordance with FD-MS is shown in the following.

FD-MS calcd. for C₃₄H₂₃N₃=473; found: m/z=473 (M⁺, 100)

### Synthesis Example 2 (Synthesis of Compound (2))

The route of synthesis of Compound (2) is shown in the following.

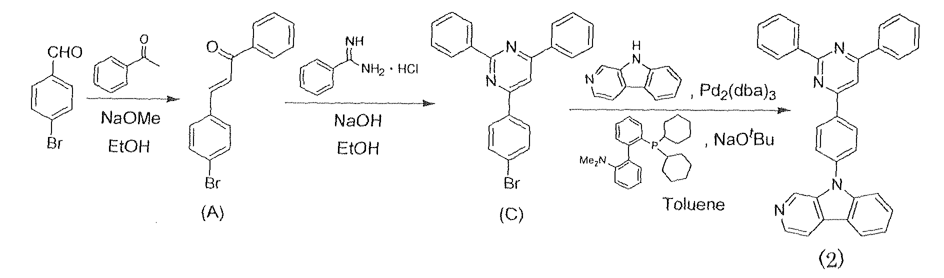

### (1) Synthesis of intermediate product (C)

Into 75 ml of ethanol, 10.0 g (35 mmole) of intermediate product (A) and 5.5 g (35 mmole) of benzamidine hydrochloride were suspended. To the resultant suspension, 2.8 g (70 mmole) of sodium hydroxide was added, and the resultant mixture was heated under the refluxing condition for 18 hours. The reaction fluid was cooled to the room temperature. To the cooled fluid, 50 ml of water was added, and the resultant mixture was stirred for 1 hour. The formed crystals were separated by filtration and washed with ethanol, and 8.2 g (the yield: 61%) of intermediate product (C) was obtained.

### (2) Synthesis of Compound (2)

In accordance with the same procedures as those conducted in Synthesis Example 1 except that intermediate product (C) was used in place of intermediate product (B), 1.8 g (the yield: 45%) of crystals were obtained.

It was confirmed in accordance with 90 MHz ¹H-NMR and FD-MS that the obtained crystals were the object compound. The result of the measurement in accordance with FD-MS is shown in the following.

FD-MS calcd. for C₃₃H₂₂N₄=474; found: m/z=474 (M⁺, 100)

### Synthesis Example 3 (Synthesis of Compound (61))

The route of synthesis of Compound (61) is shown in the following.

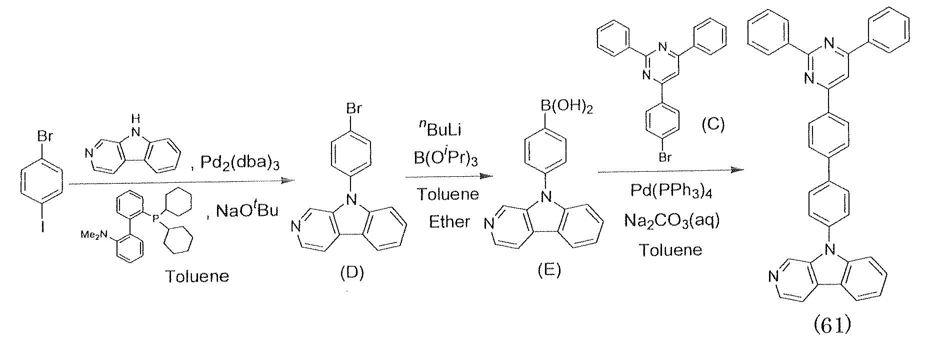

### (1) Synthesis of intermediate product (D)

Into 60 ml of toluene, 25.4 g (90 mmole) of 4-bromoiodobenzene, 10.1 g (60 mmole) of β-carboline, 0.55 g (0.6 mmole) of tris(dibenzylideneacetone)dipalladium, 0.71 g (1.8 mmole) of 2-dicyclohexylphosphino-2'-(N,N-dimethylamino)biphenyl and 8.1 g (84 mmole) of sodium tert-butoxide were suspended, and the resultant suspension was heated under the refluxing condition for 20 hours under the atmosphere of argon. The reaction fluid was cooled to the room temperature. Water was added to the fluid, and the resultant mixture was separated into two layers. The organic layer was washed with water and dried with anhydrous sodium sulfate. After the organic solvent was removed by distillation under a reduced pressure, the obtained residue was purified in accordance with the silica gel column chromatography, and 11.4 g (the yield: 59%) of crystals were obtained.

### (2) Synthesis of intermediate product (E)

Into 50 ml of toluene and 50 ml of ether, 8.1 g (25 mmole) of intermediate product (D) was dissolved. Under the atmosphere of argon, 21 ml (32 mmole) of a hexane solution of n-butyllithium (1.6 M) was added to the resultant solution at -40°C, and the obtained solution was stirred at -40 to 0°C for 1 hour. After the reaction solution was cooled to -70°C, a solution obtained by diluting 17 ml (74 mmole) of triisopropyl borate with 25 ml of ether was added dropwise. After the resultant solution was stirred at -70°C for 1 hour, the temperature was elevated to the room temperature, and the solution was stirred for 6 hours. To the resultant reaction solution, 70 ml of a 5% hydrochloric acid was added dropwise, and the obtained solution was stirred at the room temperature for 45 hours. The reaction solution was separated into two liquid layers, and the organic layer was washed with a saturated aqueous solution of sodium chloride and dried with anhydrous sodium sulfate. The organic solvent was removed by distillation under a reduced pressure until the amount of the organic solvent decreased to about one fifth of the original amount. The formed crystals were separated by filtration and washed with a mixed solvent of toluene and n-hexane and n-hexane, successively, and 3.2 g (the yield: 45%) of intermediate product (E) was obtained.

### (3) Synthesis of Compound (61)

Into 21 ml of 1,2-dimethoxyethane, 2.7 g (6.9 mmole) of intermediate product (C), 2.0 g (6.9 mmole) of intermediate product (E) and 0.16 g (0.14 mmole) of tetrakis- (triphenylphosphine)palladium were suspended. To the resultant suspension, a solution obtained by dissolving 2.2 g (21 mmole) of sodium carbonate into 11 ml of water was added, and the obtained mixture was heated under the refluxing condition for 9 hours. After the reaction fluid was separated into two layers, the organic layer was washed with a saturated solution of sodium chloride and dried with anhydrous sodium sulfate. The organic solvent was removed by distillation under a reduced pressure, and 12 ml of ethyl acetate was added to the residue. The formed crystals were separated by filtration and washed with ethyl acetate, and 2.7 g (the yield: 72%) of crystals were obtained.

It was confirmed in accordance with 90 MHz ¹H-NMR and FD-MS that the obtained crystals were the object compound. The result of the measurement in accordance with FD-MS is shown in the following.

FD-MS calcd. for C₃₉H₂₆N₄=550; found: m/z=550 (M⁺, 100)

### Synthesis Example 4 (Synthesis of Compound (68))

The route of synthesis of Compound (68) is shown in the following.

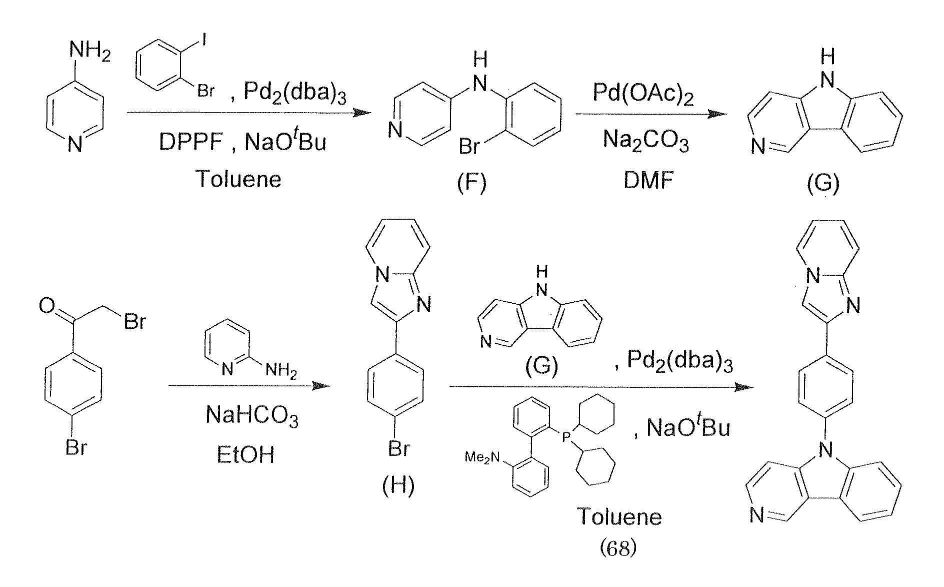

### (1) Synthesis of intermediate product (F)

Into 210 ml of toluene, 7.9 g (84 mmole) of 4-aminopyridine, 25.0 g (88 mmole) of 2-bromoiodobenzene, 1.5 g (1.6 mmole) of tris(dibenzylideneacetone)dipalladium, 1.8 g (3.2 mmole) of 1,1'-bis-(diphenylphosphino)ferrocene and 11.3 g (118 mmole) of sodium tert-butoxide were suspended, and the resultant suspension was heated under the refluxing condition for 19 hours under the atmosphere of argon. The reaction solution was cooled to the room temperature. After adding water, the reaction solution was separated into two layers, and the organic layer was washed with water and dried with anhydrous sodium sulfate. The organic solvent was removed by distillation under a reduced pressure, and 50 ml of ethanol was added to the residue. The formed crystals were separated by filtration and washed with ethanol, and 20.5 g (the yield: 98%) of intermediate product (F) was obtained.

### (2) Synthesis of intermediate product (G)

Into 80 ml of N,N-dimethylformamide, 10.0 g (40 mmole) of intermediate product (F), 0.90 g (4.0 mmole) of palladium acetate and 5.9 g (56 mmole) of sodium carbonate were suspended, and the resultant suspension was heated under the refluxing condition for 18 hours. The reaction fluid was cooled to the room temperature. After adding ethyl acetate and water, the reaction fluid was separated into two layers, and the organic layer was washed with water and a saturated solution of sodium chloride, successively, and dried with anhydrous sodium sulfate. The organic solvent was removed by distillation under a reduced pressure, and the residue was recrystallized from toluene. The obtained crystals were separated by filtration and washed with toluene, and 4.4 g (the yield: 66%) of intermediate product (G) was obtained.

### (3) Synthesis of intermediate product (H)

Into 100 ml of ethanol, 15.0 g (54 mmole) of 2,4'-dibromo-acetophenone and 5.2 g (55 mmole) of 2-aminopyridine were suspended. After 7.0 g (83 mmole) of sodium hydrogencarbonate was added, the resultant suspension was heated under the refluxing condition for 9 hours. After the reaction fluid was cooled to the room temperature, the formed crystals were separated by filtration and washed with water and ethanol, successively, and 12.5 g (the yield: 85%) of intermediate product (H) was obtained.

### (4) Synthesis of Compound (68)

In accordance with the same procedures as those conducted in Synthesis Example 1 (3) described above except that intermediate product (H) of synthesis was used in place of intermediate product (B) and intermediate product (G) was used in place of β-carboline, 1.8 g (the yield: 49%) of crystals were obtained.

It was confirmed in accordance with 90 MHz ¹H-NMR and FD-MS that the obtained crystals were the object compound. The result of the measurement in accordance with FD-MS is shown in the following.

FD-MS calcd. for C₂₄H₁₅N₄=360; found: m/z=360 (M⁺, 100)

### Synthesis Example 5 (Synthesis of Compound (72))

The route of synthesis of Compound (72) is shown in the following.

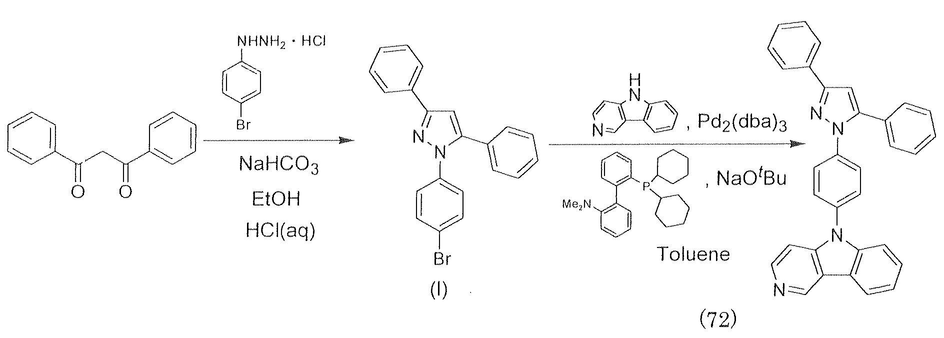

### (1) Synthesis of intermediate product (I)

Into 100 ml of ethanol, 5.0 g (22 mmole) of 4-bromophenylhydrazine hydrochloride and 1.9 g (22 mmole) of sodium hydrogencarbonate were suspended. After the resultant suspension was stirred for 1 hour, 2 ml (23 mmole) of concentrated hydrochloric acid was added, and the resultant mixture was heated under the refluxing condition for 8 hours. Then, 2 ml (23 mmole) of concentrated hydrochloric acid was added, and the obtained mixture was heated under the refluxing condition for 12 hours. After the reaction fluid was cooled to the room temperature, 2.3 ml (23 mmole) of a 30% aqueous solution of sodium hydroxide and 50 ml of water were added, and the resultant mixture was stirred for 1 hour. The formed crystals were separated by filtration and washed with ethanol, and 7.6 g (the yield: quantitative) of intermediate product (I) was obtained.

### (2) Synthesis of Compound (72)

In accordance with the same procedures as those conducted in Synthesis Example 1 (3) described above except that intermediate product (I) was used in place of intermediate product (B), 1.8 g (the yield: 46%) of crystals were obtained.

It was confirmed in accordance with 90 MHz ¹H-NMR and FD-MS that the obtained crystals were the object compound. The result of the measurement in accordance with FD-MS is shown in the following.

FD-MS calcd. for C₃₂H₂₂N₄=462; found: m/z=462 (M⁺, 100)

### Synthesis Example 6 (Synthesis of Compound (23))

The route of synthesis of Compound (23) is shown in the following.

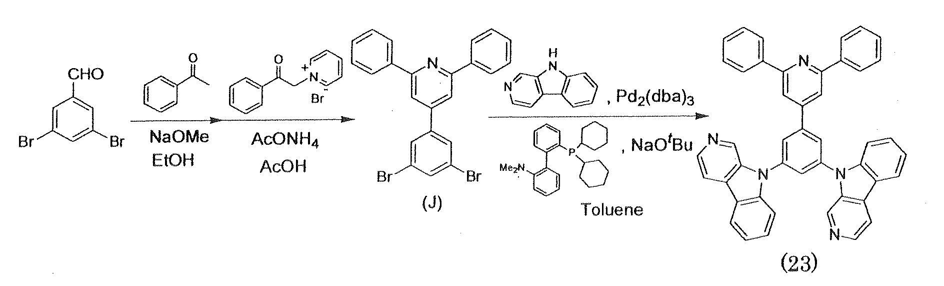

Intermediate product (J) was synthesized in accordance with the same procedures as those conducted in Synthesis Examples 1 (1) and (2) except that 3,5-dibromobenzaldehyde was used in place of 4-bromobenzaldehyde used in Synthesis Example 1 (1). Into 15 ml of toluene, 2.5 mg (5 mmole) of intermediate product (J), 1.0 g (6 mmole) of β-carboline, 0.18 g (0.2 mmole) of tris(dibenzylideneacetone)- dipalladium, 0.23 g (0.6 mmole) of 2-dicyclohexylphosphino- 2'-(N,N-dimethylamino)biphenyl and 1.0 g (11 mmole) of sodium tert-butoxide were suspended, and the resultant suspension was heated under the refluxing condition for 20 hours under the atmosphere of argon. After the reaction fluid was cooled to the room temperature, methylene chloride and water were added to the reaction fluid. The obtained fluid was separated into two layers, and the organic layer was washed with water and dried with anhydrous sodium sulfate. After the organic solvent was removed by distillation under a reduced pressure, the residue of distillation was suspended into 15 ml of toluene. To the obtained suspension, 0.18 g (0.2 mmole) of tris(dibenzylideneacetone)dipalladium, 0.23 g (0.6 mmole) of 2-dicyclohexylphosphino-2'-(N,N-dimethylamino)-biphenyl and 1.0 g (11 mmole) of sodium tert-butoxide were added, and the resultant mixture was heated under the refluxing condition for 20 hours under the atmosphere of argon. After the reaction fluid was cooled to the room temperature, methylene chloride and water were added. The obtained fluid was separated into two layers, and the organic layer was washed with water and dried with anhydrous sodium sulfate. After the organic solvent was removed by distillation under a reduced pressure, the residue was purified in accordance with the silica gel column chromatography, and 1.7 g (the yield: 53%) of crystals were obtained.

It was confirmed in accordance with 90 MHz ¹H-NMR and FD-MS that the obtained crystals were the object compound. The result of the measurement in accordance with FD-MS is shown in the following.

FD-MS calcd. for C₄₅H₂₀N₄=639; found: m/z=639 (M⁺, 100)

### Example 1 (Preparation of an organic EL device)

A glass substrate (manufactured by GEOMATEC Company) of 25 mm ×75 mm ×0.7 mm thickness having an ITO transparent electrode was cleaned by application of ultrasonic wave in isopropyl alcohol for 5 minutes and then by exposure to ozone generated by ultraviolet light for 30 minutes. The glass substrate having the transparent electrode which had been cleaned was attached to a substrate holder of a vacuum vapor deposition apparatus. On the surface of the cleaned substrate at the side having the transparent electrode, a film of copper phthalocyanine (referred to as "CuPc film", hereinafter) having a thickness of 10 nm was formed in a manner such that the formed film covered the transparent electrode. The formed CuPc film worked as the hole injecting layer. On the formed CuPc film, a film of 4,4'-bis[N- (1-naphthyl)-N-phenylamino]-biphenyl shown below (referred to as "α-NPD film", hereinafter) having a thickness of 30 nm was formed. The formed α-NPD film worked as the hole transporting layer. On the formed α-NPD film, a film of Compound (1) prepared above having a thickness of 30 nm was formed by vapor deposition using Compound (1) as the host material, and the light emitting layer was formed. At the same time, tris(2-phenylpyridine)Ir shown below (referred to as "Ir(ppy)₃", hereinafter) was added as the Ir metal complex emitting phosphorescent light. The content of Ir(ppy)₃ in the light emitting layer was 5% by weight. This film worked as the light emitting layer. On the film formed above, a film of (1,1'-bisphenyl)-4-olato)bis(2-methyl-8-quinolinolato)aluminum shown below (referred to as "BAlq film", hereinafter) having a thickness of 10 nm was formed. BAlq film worked as the hole barrier layer. On this film, a film of an aluminum complex of 8-hydroxyquinoline shown below (referred to as "Alq film", hereinafter) having a thickness of 40 nm was formed. Alq film worked as the electron injecting layer. Then, LiF which is an alkali metal halide was vapor deposited to form a film having a thickness of 0.2 nm. On the formed film, aluminum was vapor deposited to form a film having a thickness of 150 nm. The Al/LiF film worked as the cathode. An organic EL device was prepared in the manner described above.

The triplet energy and the singlet energy of the host material used in the light emitting layer were measured in accordance with the methods described above in (1) and (2), respectively. The results are shown in Table 1.

The device prepared above was examined by passing electric current. Green light was emitted at a luminance of 99 cd/m² under a voltage of 5.2 V and a current density of 0.26 mA/cm². The chromaticity coordinates were (0.32, 0.62), and the current efficiency was 38.6 cd/A. These results are shown in Table 1.

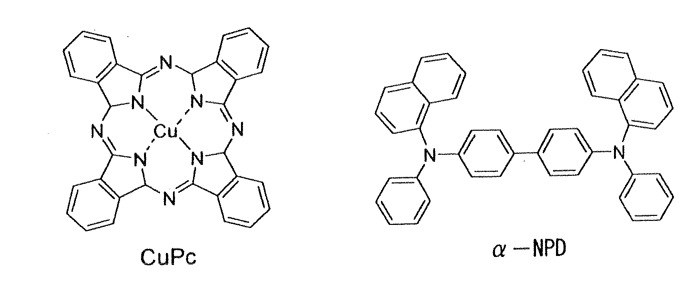

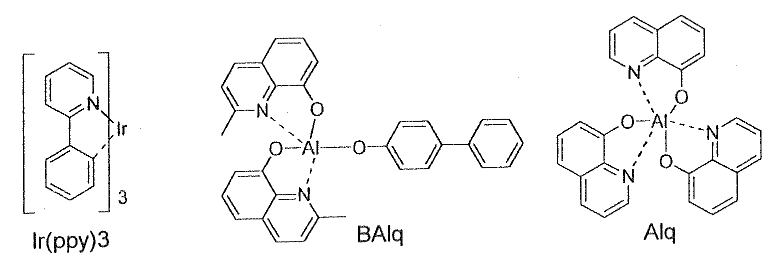

### Examples 2 and 3

Organic EL devices were prepared in accordance with the same procedures as those conducted in Example 1 except that compounds shown in Table 1 were used in place of Compound (1), and the triplet energy, the singlet energy, the voltage, the current density, the luminance, the current efficiency and the chromaticity were measured in accordance with the same methods as those conducted in Example 1. The results are shown in Table 1.

### Comparative Example 1

An organic EL device was prepared in accordance with the same procedures as those conducted in Example 1 except that compound (BCz) shown in the following was used in place of Compound (1), and the triplet energy, the singlet energy, the voltage, the current density, the luminance, the current efficiency and the chromaticity were measured in accordance with the same methods as those conducted in Example 1. The results are shown in Table 1.

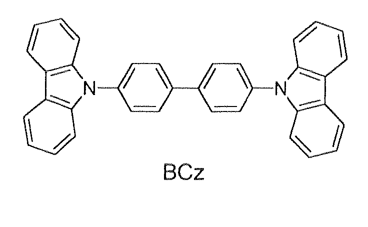

### Comparative Example 2

An organic EL device was prepared in accordance with the same procedures as those conducted in Example 1 except that compound (A-10) shown in the following, which is described in the specification of the United States Patent Application Publication No. 2002-28329, was used in place of Compound (1), and the properties were measured in accordance with the same methods as those conducted in Example 1. The results are shown in Table 1.

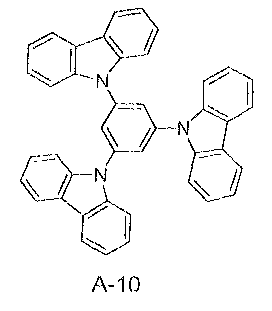

**Table 1 - 1**

| | Host material of light emitting layer | Triplet energy | Singlet energy | Voltage | Current density |
|---|---|---|---|---|---|
| | | (eV) | (eV) | (V) | (mA/cm²) |
| Example 1 | (1) | 2.8 | 3.4 | 5.2 | 0.26 |
| Example 2 | (61) | 2.6 | 3.3 | 5.5 | 0.24 |
| Example 3 | (68) | 2.7 | 3.5 | 5.6 | 0.27 |
| Comparative Example 1 | (BCz) | 2.8 | 3.6 | 5.4 | 0.31 |
| Comparative Example 2 | (A-10) | 3.1 | 3.7 | 5.9 | 0.32 |

**Table 1 - 2**

| | Luminance | Current efficiency | Chromaticity coordinates | Color of emitted light |
|---|---|---|---|---|
| | (cd/m²) | (cd/A) | (x, y) | |
| Example 1 | 99 | 38.6 | (0.32,0.62) | green |
| Example 2 | 102 | 42.8 | (0.32, 0.61) | green |
| Example 3 | 100 | 37.2 | (0.32, 0.61) | green |
| Comparative Example 1 | 101 | 32.6 | (0.32, 0.61) | green |
| Comparative Example 2 | 100 | 31.8 | (0.32, 0.61) | green |

As shown in Table 1, the organic EL devices using the material for organic EL devices of the present invention emitted green light with greater efficiencies than those of devices of Comparative Examples 1 and 2 in which conventional compounds (BCz and A-10, respectively) were used. Since the material for organic EL devices of the present invention had a great energy gap, the light emitting molecule having a great energy gap could be mixed into the light emitting layer and used for the light emission.

### Example 4

A glass substrate (manufactured by GEOMATEC Company) of 25 mm × 75 mm × 0.7 mm thickness having an ITO transparent electrode was cleaned by application of ultrasonic wave in isopropyl alcohol for 5 minutes and then by exposure to ozone generated by ultraviolet light for 30 minutes. The glass substrate having the transparent electrode which had been cleaned was attached to a substrate holder of a vacuum vapor deposition apparatus. On the surface of the cleaned substrate at the side having the transparent electrode, CuPc film having a thickness of 10 nm was formed in a manner such that the formed film covered the transparent electrode. The formed CuPc film worked as the hole injecting layer. On the formed CuPc film, a film of 1,1'-bis[4-N,N-di(para-tolyl)aminophenyl]cyclohexane shown below (referred to as "TPAC film", hereinafter) having a thickness of 30 nm was formed. The formed TPAC film worked as the hole transporting layer. On the formed TPAC film, a film of Compound (1) prepared above having a thickness of 30 nm was formed by vapor deposition, and the light emitting layer was formed. At the same time, Ir bis[(4,6-difluorophenyl)pyridinato-N,C²']picolinate shown below (referred to as "FIrpic", hereinafter) was added as the Ir metal complex emitting phosphorescent light. The content of FIrpic in the light emitting layer was 7% by weight. This film worked as the light emitting layer. On the film formed above, Alq film having a thickness of 30 nm was formed. Alq film worked as the electron injecting layer. Then, LiF which is an alkali metal halide was vapor deposited to form a film having a thickness of 0.2 nm. On the formed film, aluminum was vapor deposited to form a film having a thickness of 150 nm. The Al/LiF film worked as the cathode. An organic EL device was prepared in the manner described above.

The triplet energy and the singlet energy of the host material used in the light emitting layer were measured in accordance with the methods described above in (1) and (2), respectively. The results are shown in Table 2.

The device prepared above was examined by passing electric current. Blue light was emitted at a luminance of 101 cd/m² under a voltage of 6.4 V and a current density of 0.65 mA/cm². The chromaticity coordinates were (0.17, 0.39), and the current efficiency was 15.6 cd/A.

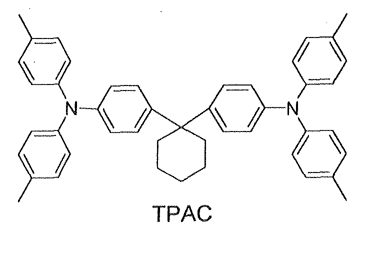

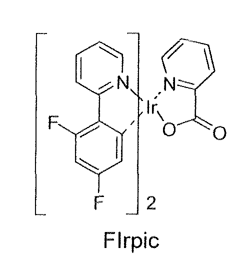

### Examples 5 and 6

Organic EL devices were prepared in accordance with the same procedures as those conducted in Example 4 except that compounds shown in Table 2 were used in place of Compound (1), and the triplet energy, the singlet energy, the voltage, the current density, the luminance, the current efficiency and the chromaticity were measured in accordance with the same methods as those conducted in Example 2. The results are shown in Table 2.

### Comparative Example 3

An organic EL device was prepared in accordance with the same procedures as those conducted in Example 4 except that compound (BCz) shown above was used in place of Compound (1), and the triplet energy, the singlet energy, the voltage, the current density, the luminance, the current efficiency and the chromaticity were measured in accordance with the same methods as those conducted in Example 4. The results are shown in Table 2.

### Comparative Example 4

An organic EL device was prepared in accordance with the same procedures as those conducted in Comparative Example 3 except that compound (α-NPD) shown above was used for the hole transporting layer in place of TPAC and compound (BAlq) shown above was used for the electron injecting layer in place of compound (Alq). The triplet energy, the singlet energy, the voltage, the current density, the luminance, the current efficiency and the chromaticity were measured in accordance with the same methods. The results are shown in Table 2.

**Table 2 - 1**

| | Host material of light emitting layer | Triplet energy | Singlet energy | Voltage | Current density |
|---|---|---|---|---|---|
| | | (eV) | (eV) | (V) | (mA/cm²) |
| Example 4 | (1) | 2.8 | 3.4 | 6.4 | 0.65 |
| Example 5 | (2) | 2.8 | 3.4 | 6.8 | 0.57 |
| Example 6 | (3) | 2.7 | 3.2 | 6.9 | 0.73 |
| Comparative Example 3 | (BCz) | 2.8 | 3.6 | 7.8 | 1.70 |
| Comparative Example 4 | (BCz) | 2.8 | 3.6 | 7.6 | 1.09 |

**Table 2 - 2**

| | Luminance | Current efficiency | Chromaticity coordinates light | Color of emitted |
|---|---|---|---|---|
| | (cd/m²) | (cd/A) | (x, y) | |
| Example 4 | 101 | 15.6 | (0.17,0.39) | blue |
| Example 5 | 103 | 18.2 | (0.18, 0.39) | blue |
| Example 6 | 97 | 13.3 | (0.18, 0.39) | blue |
| Comparative Example 3 | 98 | 5.8 | (0.16, 0.37) | blue |
| Comparative Example 4 | 99 | 9.2 | (0.17, 0.37) | blue |

As shown in Table 2, the organic EL devices using the material for organic EL devices of the present invention could be driven under a lower voltage and emitted blue light with greater efficiencies than those of devices of Comparative Example 3 and 4 in which conventional compound (BCz) was used. Since the material for organic EL devices of the present invention had a great energy gap, the light emitting molecule having a great energy gap could be mixed into the light emitting layer and used for the light emission.

### INDUSTRIAL APPLICABILITY

As described specifically in the above, the organic EL device which emits light under a low voltage with a greater current efficiency by utilizing the emission of phosphorescent light can be obtained when the material for organic EL devices of the present invention comprising the compound represented by general formula (1) is used. Therefore, the organic EL device of the present invention is very useful for applications such as light sources of various electronic instruments.

## Claims

1. A material for organic electroluminescence devices which comprises a compound represented by following general formula (1): 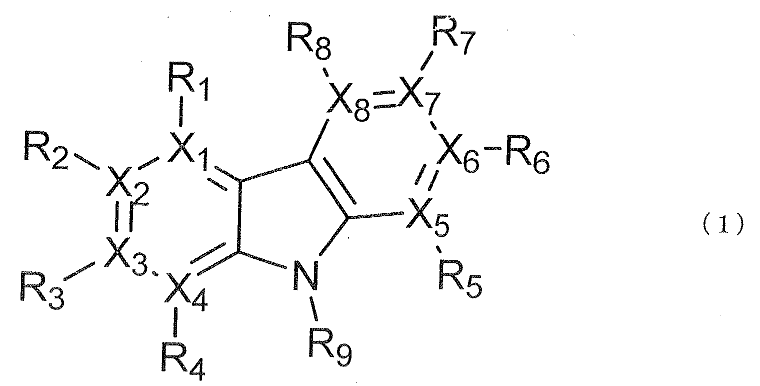 wherein X₁ to X₈ each represent carbon atom or nitrogen atom, and at least one of X₁ to X₈ represents nitrogen atom; when any of X₁ to X₈ represent carbon atom, R₁ to R₈ connected to X₁ to X₈ representing carbon atom, respectively, each represent a substituent bonded to the carbon atom; adjacent substituents represented by R₁ to R₈ may form a ring; when any of X₁ to X₈ represent nitrogen atom, R₁ to R₈ connected to X₁ to X₈ representing nitrogen atom, respectively, each represent a noncovalent electron pair; and R₉ represents a substituent.

2. A material for organic electroluminescence devices according to Claim 1, wherein R₁ to R₉ each represent -L or -L-Y, wherein
L represents hydrogen atom, a substituted or unsubstituted aryl group having 6 to 40 carbon atoms, a substituted or unsubstituted heterocyclic group having 2 to 40 carbon atoms, a substituted or unsubstituted linear or branched alkyl group having 1 to 20 carbon atoms, a substituted or unsubstituted cycloalkyl group having 6 to 40 carbon atoms, a substituted or unsubstituted amino group having 2 to 40 carbon atoms, a substituted or unsubstituted linear or branched alkoxyl group having 1 to 40 carbon atoms, a halogen atom, nitro group, a substituted or unsubstituted arylene group having 6 to 40 carbon atoms, a substituted or unsubstituted divalent heterocyclic group having 2 to 40 carbon atoms, a linear or branched substituted or unsubstituted alkylene group having 1 to 20 carbon atoms or a substituted or unsubstituted cycloalkylene group having 6 to 40 carbon atoms; and
Y represents hydrogen atom, a substituted or unsubstituted aryl group having 6 to 40 carbon atoms, a substituted or unsubstituted heterocyclic group having 2 to 40 carbon atoms, a substituted or unsubstituted linear or branched alkyl group having 1 to 20 carbon atoms, a substituted or unsubstituted cycloalkyl group having 6 to 40 carbon atoms, a substituted or unsubstituted amino group having 2 to 40 carbon atoms, a substituted or unsubstituted linear or branched alkoxyl group having 1 to 40 carbon atoms, a halogen atom or nitro group.

3. A material for organic electroluminescence devices according to Claim 1, wherein one to three among X₁ to X₈ each represent nitrogen atom, and the others each represent carbon atom.

4. A material for organic electroluminescence devices according to Claim 1, wherein at least one of X₃ and X₆ among X₁ to X₈ represents nitrogen atom, and the others each represent carbon atom.

5. A material for organic electroluminescence devices according to Claim 1, wherein at least one of R₁ to R₈ represents β-carbolinyl group.

6. A material for organic electroluminescence devices according to Claim 2, wherein at least one of L and Y represents β-carbolinyl group.

7. A material for organic electroluminescence devices according to Claim 1, wherein an energy gap of a triplet state is 2.5 to 3.3 eV.

8. A material for organic electroluminescence devices according to Claim 1, wherein an energy gap of a singlet state is 2.8 to 3.8 eV.

9. An organic electroluminescence device comprising a cathode, an anode and an organic thin film layer which is sandwiched between the cathode and the anode and comprises at least one layer, wherein at least one layer in the organic thin film layer contains a material for organic electroluminescence devices described in Claim 1.

10. An organic electroluminescence device comprising a cathode, an anode and an organic thin film layer which is sandwiched between the cathode and the anode and comprises at least one layer, wherein a light emitting layer contains a material for organic electroluminescence devices described in Claim 1.

11. An organic electroluminescence device comprising a cathode, an anode and an organic thin film layer which is sandwiched between the cathode and the anode and comprises at least one layer, wherein at least one of an electron transporting layer and an electron injecting layer contains a material for organic electroluminescence devices described in Claim 1.

12. An organic electroluminescence device comprising a cathode, an anode and an organic thin film layer which is sandwiched between the cathode and the anode and comprises at least one layer, wherein at least one of a hole transporting layer and a hole injecting layer contains a material for organic electroluminescence devices described in Claim 1.

13. An organic electroluminescence device according to any one of Claims 9 to 12, wherein the material for organic electroluminescence devices is an organic host material.

14. An organic electroluminescence device according to any one of Claims 9 to 12, which comprises an inorganic compound layer sandwiched between at least one of the electrodes and the organic thin film layer.

15. An organic electroluminescence device according to any one of Claims 9 to 12, wherein the organic thin film layer contains a phosphorescent emissive compound.

16. An organic electroluminescence device according to any one of Claims 9 to 12, which emits bluish light.
